Europäisches Patentamt

European Patent Office

Office européen des brevets

(19)

(11) Veröffentlichungsnummer: **0 158 599**
**B1**

(12) **EUROPÄISCHE PATENTSCHRIFT**

(45) Veröffentlichungstag der Patentschrift: 10.10.90

(21) Anmeldenummer: **85810152.0**

(22) Anmeldetag: **03.04.85**

(51) Int. Cl.⁵: **C 12 P 21/00,** C 12 N 15/00,
C 12 N 5/00, G 01 N 33/577,
G 01 N 33/573, C 12 N 9/64,
A 61 K 39/395 // (C12P21/00,
C12R1:91)

(54) Neue monoklonale Antikörper und Hybridoma-Zellen, Verfahren zu ihrer Herstellung und ihre Anwendungen.

(30) Priorität: **09.04.84 CH 1781/84**

(43) Veröffentlichungstag der Anmeldung:
**16.10.85 Patentblatt 85/42**

(45) Bekanntmachung des Hinweises auf die
Patenterteilung:
**10.10.90 Patentblatt 90/41**

(84) Benannte Vertragsstaaten:
**AT BE CH DE FR GB IT LI LU NL SE**

(56) Entgegenhaltungen:
**CHEMICAL ABSTRACTS, Band 100, Nr. 1,
02.01.1984, Seite 852, Ref. Nr. 846p; Columbus,
Ohio, US; V.J.DZAU et al.:"Antibodies as
specifice renin inhibitors: studies with
polyclonal and monoclonal antibodies and Fab
fragments"**

**BIOLOGICAL ABSTRACTS, Band 79, Nr. 5, 1985,
Seite 310, Ref. Nr. 39436; Philadelphia, US; F.X.
GALEN et al.:"New monoclonal antibodies
directed against human renin. Powerful tools
for the investigation of the renin system"**

(73) Patentinhaber: **CIBA-GEIGY AG
Klybeckstrasse 141
CH-4002 Basel (CH)**

(72) Erfinder: **Heusser, Christoph, Dr.
Im Bertschenacker 21
CH-4103 Bottmingen (CH)**
Erfinder: **Andreatta, Rudolf Heinrich, Dr.
Ochsengasse 32
CH-4123 Allschwil (CH)**
Erfinder: **Alkan, Sefik, Prof. Dr.
Binsenackerstrasse 3
CH-4125 Riehen (CH)**
Erfinder: **Wood, Jeanette, Dr.
Mühleweg 35
CH-4105 Biel-Benken (CH)**

(56) Entgegenhaltungen:
CHEMICAL ABSTRACTS, Band 102, Nr. 13,
01.04.1985, Seite 291, Ref. Nr. 108589n;
Columbus, Ohio, US; P. CORVOL et
al.:"Monoclonal antibodies to human renin"

CHEMICAL ABSTRACTS, Band 100, Nr. 5,
30.01.1984, Seite 175, Ref. Nr. 31213j;
Columbus, Ohio, US; F.X. GALEN et
al.:"Application of human rening antibodies"

## Beschreibung

Die Erfindung betrifft neue monoklonale Antikörper mit hoher Affinität zu Human-Renin, Derivate davon, Verfahren zur Herstellung dieser Antikörper und ihrer Derivate, Hybridoma-Zellinien, die diese Antikörper produzieren, Verfahren zur Herstellung besagter Hybridoma-Zellinien, ferner die Verwendung der monoklonalen Antikörper und ihrer Derivate zur qualitativen und quantitativen Bestimmung von Human-Renin und strukturell ähnlichem Renin, Test-Kits dazu, die Verwendung der monoklonalen Antikörper und ihrer Derivate zur Reinigung von Human-Renin und strukturell ähnlichem Renin, pharmazeutische Präparate enthaltend besagte Antikörper oder Derivate davon und die Verwendung der monoklonalen Antikörper und ihrer Derivate zur Behandlung von Bluthochdruck und Herzinsuffizienz.

Hintergrund der Erfindung

Renin ist ein proteolytisches Enzym, das durch Freisetzung der Angiotensine mitverantwortlich ist für die Regulierung des Blutdruckes. Human-Renin ist ein glykosyliertes Protein mit einem Molekulargewicht von ca. 40'000. Einige strukturelle Eigenschaften von Human-Renin sind bekannt. Beispielsweise ist die Aminosäure-Sequenz des Polypeptids durch Bestimmung der für Renin codierenden komplementären Desoxyribonucleinsäure (cDNA) aufgeklärt worden.

Renin wird in den Nieren aus einer inaktiven Form, dem Pro-Renin, freigesetzt und gelangt dann in die Blutbahn, wo es in Konzentrationen von 20—100 pg/ml zu finden ist. Dort bewirkt es die Spaltung von Angiotensinogen (Renin-Substrat) unter Bildung des Dekapeptids Angiotensin I, das seinerseits vom sogenannten "Angiotensinumwandelnden Enzym" (Angiotensin converting enzyme, ACE) in der Lunge, den Nieren und anderen Organen zum Oktapeptid Angiotensin II gespalten wird. Angiotensin II erhöht den Blutdruck sowohl direkt durch arterielle Konstriktion als auch indirekt durch Freisetzung des Natrium zurückhaltenden Hormons Aldosteron aus den Nebennieren, womit ein Anstieg des extrazellulären Flüssigkeitsvolumens verbunden ist. Angiotensin II wird durch Angiotensinasen zum Heptapeptid Angiotensin III, das ähnliche Wirkungen wie Angiotensin II zeigt, und zu kleineren, inaktiven Bruckstücken abgebaut.

Die Behandlung von gewissen Formen von Bluthochdruck (Hypertension) und Herzinsuffizienz ist durch eine Beeinflussung des Renin-Angiotensin-Systems möglich. Dabei kann eine Blutdrucksenkung erreicht werden durch (a) Hemmung der Wirkung von Angiotensin II (oder III) auf seine Rezeptoren, (b) Hemmung des Angiotensin-umwandelnden Enzyms oder (c) Hemmung der Wirkung von Renin auf Angiotensinogen. Renin-Hemmer sind beispielsweise Pepstatin und Pepstatin-Analoge, Angiotensinogen-Analoge und Renin-Antikörper. Solche Renin-Hemmer vermindern die Freisetzung von Angiotensin I aus Angiotensinogen und verringern damit auch die Konzentration des aktiven Peptidhormons Angiotensin II. Gegenüber den bekannten Renin-Hermmern haben die erfindungsgemässen Renin-Antikörper den Vorteil, schon in extrem geringen Konzentrationen und ganz spezifisch die Wirkung des Renins zu hemmen.

Die Verwendung von Antikörpern in Diagnose und Therapie war bis vor kurzem in ihrem Anwendungsbereich stark eingeschränkt. Antikörper wurden in geringen Mengen aus tierischem Serum als komplexe Mischung verschiedener Proteine gewonnen. Standardisierung der Antikörper war nicht möglich, weil jedes immunisierte tierische Individuum und auch ein einzelnes Individuum bei wiederholter Immunisierung jeweils ein Serum mit Antikörpern verschiedener Zusammensetzung ergibt. Durch Anwendung einer von Köhler und Milstein [1] entwickelten neuen Technik ist es nun möglich geworden, Antikörper in homogener Form, d.h. sogenannte monoklonale Antikörper, reproduzierbar in theoretisch unbeschränkten Mengen aus Zellkulturen zu gewinnen. Durch Fusion von geeigneten Myeloma-Zellen mit Antikörper-produzierenden Lymphozyten aus einem mit Antigen Immunisierten Spender entstehen Hybridoma-Zellen, die die Fähikgkeit zur unbegrenzten Zellteilung und zu unbegrenztem Wachstum in vitro mit der Produktion eines homogenen Antikörpers verbinden. Somit ist es möglich, die Immunantwort eines Organismus auf ein bestimmtes Antigen zu verselbständigen und monoklonale Antikörper durch permanentes Kultivieren von Hybridzellen herzustellen.

Obwohl bis heute viele Beispiele für die Herstellung spezifischer monoklonaler Antikörper durch Hybridoma-Technik bekannt geworden sind und das allgemeine Vorgehen prinzipiell beschrieben ist, so treten doch bei jedem neuen Beispiel spezifische Probleme auf, die eine Anpassung der Technik an den gegebenen Fall erfordern. Ohne solche Anpassung gibt es keine Gewissheit, dass die gewünschten Hybridoma-Zellen je gebildet werden, dass sie genetisch stabil sind, die gewünschten monoklonalen Antikörper herstellen und dass die dermassen hergestellten Antikörper die gewünschte Spezifität aufweisen. Der Grad des Erfolges wird im Prinzip beeinflusst durch Art und Reinheit des für die Immunisierung des Lymphozyten-Spenders verwendeten Antigens, die Immunisierungsmethode, die Technik der Zell-Fusion, das Vorgehen bei der Selektion geeigneter Hybridoma-Zellinien und die Art und Weise der Isolierung und Reinigung der monoklonalen Antikörper.

Monoklonale Antikörper, die Human-Renin binden, sind bekannt. Simon et al. [2] beschreiben einen monoklonalen Antikörper mit niedriger Affinität für Human-Renin. Dzau et al. [3] beschreiben monoklonale Antikörper, die Human-Renin binden und gleichzeitig dessen enzymatische Funktion hemmen. Zur Bestimmung von Human-Renin in Blut werden grundsätzlich zwei verschiedene Methoden angewendet [4]. Einerseits wird die enzymatische Aktivität von Human-Renin in Plasma durch Bestimmung der aus Angiotensionogen freigesetzten Menge Angiotensin I gemessen. Eine Mass für die Gesamtmenge Renin ergibt sich dann durch Bestimmung der enzymatischen Aktivität nach Aktivierung von inaktivem Plasma-Renin, beispielsweise durch Behandlung mit Säure bei pH 3 bis 4. Diese indirekte Methode erlaubt zwar die Messung von Renin bei niedrigen Konzentrationen, ist jedoch mit grossen Unsicherheiten behaftet. Andrerseits wird Human-Renin in Immunoassays mit Hilfe der bekannten monoklonalen Antikörper oder mit polyklonalen Antikörpern aus Serum bestimmt. Diese direkte Methode weist aber bisher keine genügende Empfindlichkeit auf, um die ausserordentlich niedrigen Konzentrationen von Human-Renin in Plasma zuverlässig zu messen. Es besteht deshalb ein Bedürfnis für monoklonale Antikörper, die Human-Renin wesentlich stärker binden als die bekannten Antikörper, damit diese in Immunoassays eingesetzt werden können, um Renin im menschlichen Blutplasma genau zu bestimmen. Ferner besteht ein Bedürfnis für monoklonale Antikörper, die die Wirkung des Enzyms Renin so effizient hemmen, dass sie zur Therapie von Bluthochdruck verwendet werden können. Die vorliegende Erfindung stellt eine Lösung dieser Aufgabe dar, indem sie monoklonale Antikörper bereitstellt, die Human-Renin stark binden und gleichzeitig dessen enzymatische Funktion effizient hemmen.

Beschreibung der Erfindung

Die Erfindung betrifft monoklonale Antikörper gegen Human-Renin und strukturell ähnliches Renin, sowie Derivate davon, gekennzeichnet durch eine hohe Affinität zu Human-Renin.

Monoklonale Antikörper gegen Human-Renin können charakterisiert werden durch die Bindungs-Konstanten an Oberflächen-gebundenes oder gelöstes Human-Renin, die Hemm-Konzentration der Enzym-Aktivität von Human-Renin, die Kreuzreaktivität gegebüber verwandten Antigenen, beispielsweise gegenüber Renin anderer Spezies, die Immunglobulin-Klasse oder Subklasse, sowie die N-terminale Aninosäuresequenz der Polypeptidketten.

Die Erfindung betrifft insbesondere monoklonale Antikörper und Derivate davon, die bei einer Konzentration von $2 \times 10^{-8}$M die Enzym-Aktivität von Human-Renin zu mindestens 50% hemmen, und monoklonale Antikörper und Derivate davon, die zusätzlich bei einer Konzentration von $3 \times 10^{-10}$M mindestens 50% gelöstes Human-Renin, das in limitierender Menge zugesetzt ist, binden. Bevorzugt sind monoklonale Antikörper und Derivate davon, die bei einer Konzentration von $2 \times 10^{-9}$M die Enzym-Aktivität von Human-Renin zu mindestens 50% hemmen. Ganz besonders bevorzugt sind monoklonale Antikörper und Derivate davon, die bei einer Konzentration von $5 \times 10^{-11}$M die Enzym-Aktivität von Human-Renin zu mindestens 50% hemmen.

Ein Beispiel für ein ganz besonders bevorzugter monoklonaler Antikörper ist der Antikörper mit der Bezeichnung R 3—36—16, der von der Zellinie R 3—36—16 produziert wird. Monoklonale Antikörper R 3—36—16 sind Gamma 1 Kappa-Immunglobuline, die in der ersten hypervariablen Region der leichten Polypeptidkette in Position 28—38 von N-Terminus die Aminosäuren $^{28}$Serin-$^{29}$Valin, $^{31}$Serin-$^{32}$Tyrosin-$^{33}$Glycin-$^{34}$Lysin und $^{36}$Phenylalanin-$^{37}$Methionin enthalten.

Erfindungsgemässe Derivate von monoklonalen Antikörpern sind beispielsweise Bruchstücke, wie Fab, Fab' oder F(ab')$_2$-Fragmente, die ihre Spezifität für die antigenen Determinanten von Human-Renin beibehalten, radioaktiv markierte monoklonale Antikörper, die beispielsweise mit radioaktivem Iod ($^{125}$I, $^{131}$I), Kohlenstoff ($^{14}$C), Schwefel ($^{35}$S), Tritium ($^{3}$H) oder dergleichen markiert sind, oder monoklonale Antikörper-Konjugate mit Enzymen wie Meerrettich-Peroxidase, alkalische Phosphatase, β-D-Galactosidase, Glucose-Oxidase, Glucoamylase, Carboanhydrase, Acetylchlolinesterase, Lysozym, Malat-Dehydrogenase oder Glucose-6-phosphat-Dehydrogenase.

Bevorzugte Derivate sind mit $^{125}$Iod markierte monoklonale Antikörper und monoklonale Antikörper-Konjugate mit alkalischer Phosphatase.

Die erfindungsgemässen monoklonalen Antikörper und Derivate davon werden nach an sich bekannten Verfahren erhalten, indem man solche monoklonale Antikörper produzierende Hybridoma-Zellen

a) in vitro kultiviert und aus den Kulturüberständen die monoklonalen Antikörper isoliert, oder

b) in vivo in einen geeigneten Säugetier vermehrt und aus den Körperflüssigkeiten dieses Säuegetiers die monoklonalen Antikörper isoliert, und, wenn erwünscht,

c) einen erhaltenen monoklonalen Antikörper in ein Derivat davon überführt.

Geeignete Kulturmedien für die in vitro Kultivierung nach Verfahren a) sind die üblichen Standard-Kulturmedien, beispielsweise Dulbecco's Modified Eagle-Medium oder RPMI 1640-Medium ergänzt mit fötalem Kälber-Serum. Zur Isolierung der monoklonalen Antikörper werden die Proteine in den Kulturüberständen mit Ammoniumsulfat oder dergleichen gefällt und mit den üblichen chromatographischen Methoden, beispielsweise Gel-Filtration, Ionenaustauscherchromatographie, Chromatographie an DEAE-Cellulose oder Immuno-Affinitätschromatographie, gereinigt.

Grosse Mengen der gewünschten monoklonalen Antikörper können erhalten werden durch Vermehrung der Hybridoma-Zellen in vivo gemäss Verfahren b). Dazu werden Zellklone in syngene Säugetiere injiziert und nach 1—3 Wochen die Antikörper aus den Körperflüssigkeiten dieser Säuger isoliert. Beispielsweise werden Hybridoma-Zellen abstammend von Balb/c-Mäusen intraperitoneal in gegebenenfalls mit einem Kohlenwasserstoff wie Pristan vorbehandelte Balb/c-Mäuse injiziert und nach 8—10 Tagen diesen Tieren Aszitesflüssigkeit entnommen. Die gewünschten monoklonalen Antikörper werden aus den Körperflüssigkeiten nach an sich bekannten Methoden isoliert, beispielsweise durch Fällung mit Ammoniumsulfat oder dergleichen und chromatographische Reinigung z.B. über DEAE-

4

EP 0 158 599 B1

Cellulose, Ionenaustauscherharz, durch Gel-Filtration oder Immuno-Affinitätschromatographie.

Erfindungsgemässe Bruchstücke von monoklonalen Antikörpern, beispielsweise Fab, Fab' oder F(ab')$_2$-Fragmente, die ihre Spezifität für die antigenen Determinanten von Human-Renin beibehalten, werden nach an sich bekannten Methoden hergestellt, beispielsweise durch Behandlung von nach Verfahren a) oder b) hergestellten monoklonalen Antikörpern mit Enzymen wie Pepsin oder Papain und/ oder Spaltung von Disulfid-Bindungen durch chemische Reduktion.

Mit Iod ($^{125}$I, $^{131}$I) radioaktiv markierte monoklonale Antikörper werden aus den erfindungsgemässen monoklonalen Antikörpern durch an sich bekannte Iodierung, beispielsweise mit radioaktivem Natrium- oder Kaliumiodid und einem chemischen Oxidationsmittel, wie Natriumhypochlorit, Chloramin T oder dergleichen, oder einem enzymatischen Oxidationsmittel, wie Lactoperoxidase, Glucoseoxidase und Glucose, erhalten. Erfindungsgemässe radioaktiv markierte monoklonale Antikörper werden auch hergestellt, indem man auf an sich bekannte Weise den Kulturmedien für die in vitro-Kultivierung radioaktiv markierte Nährstoffe enthaltend radioaktiven Kohlenstoff ($^{14}$C), Tritium ($^3$H), Schwefel ($^{35}$S) oder dergleichen, beispielsweise L-($^{14}$C)-Leucin, L-($^3$H)-Leucin oder L-($^{35}$S)-Methionin, zugibt und die monoklonalen Antikörper nach Verfahren a) gewinnt.

Enzym-markierte Erfindungsgemässe monoklonale Antikörper erhält man nach an sich bekannten Methoden, indem man nach Verfahren a) oder b) hergestellte monoklonale Antikörper und das gewünschte Enzym mit einem Kupplungsreagens, beispielsweise Glutaraldehyd, Periodat, N,N'-o-Phenylendimaleimid, N-(m-Maleimidobenzoyloxy)-succinimid, N-(3-(2'-Pyridyldithio)-propionoxy)-succinimid oder dergleichen, umsetzt.

Die Erfindung betrifft ferner Hybridoma-Zellinien, dadurch gekennzeichnet, dass sie monoklonale Antikörper mit hoher Affinität zu Human-Renin produzieren. Insbesondere betrifft die Erfindung Zellinien, die monoklonale Antikörper produzieren, die bei einer Konzentration von $2\times10^{-8}$M die Enzym-Aktivität von Human-Renin zu mindestens 50% hemmen. Bevorzugt sind Zellinien, die monoklonale Antikörper produzieren, die zusätzlich bei einer Konzentration von $3\times10^{-10}$M (Mol/Liter) mindestens 50% Human-Renin, das in limitierender Menge zugesetzt ist, binden. Besonders bevorzugt sind Zellinien, die monoklonale Antikörper produzieren, die bei einer Konzentration von $2\times10^{-9}$M die Enzym-Aktivität von Human-Renin zu mindestens 50% hemmen. Ganz besonders bevorzugt sind Zellinien, die monoklonale Antikörper produzieren, die bei einer Konzentration von $5\times10^{-11}$M die Enzym-Aktivität von Human-Renin zu mindestens 50% hemmen.

Ein Beispiel für eine ganz besonders bevorzugte Hybridoma-Zellinie, die monoklonale Antikörper hoher Affinität produziert, ist die Zellinie mit der Bezeichnung R 3—36—16, die am 7.11.1983 in der "Collection Nationale de Cultures de Microorganismes" des Institut Pasteur in Paris unter der Nummer I—253 hinterlegt worden ist. Hybridoma-Zellinie R 3—36—16 ist ein Hybrid der Maus-Myeloma-Zellinie Sp2/0-Ag14 und eines B-Lymphozyten der Milz einer Balb/c-Maus. R 3—36—16 ist eine stabile Zellinie, die monoklonale Antikörper gleichbleibender Spezifität ausscheidet und aus tiefgefrorenen Kulturen durch Auftauen und Re-Klonierung aktiviert werden kann.

Die Erfindung betrifft ferner ein Verfahren zur Herstellung von Hybridoma-Zellen, die monoklonale Antikörper mit hoher Affinität zu Human-Renin produzieren, dadurch gekennzeichnet, dass man geeignete Säugetiere mit gereinigtem Human-Renin immunisiert, dem Säugetier entnommene Antikörper-produzierende Zellen mit Myeloma-Zellen fusioniert, die erhaltenen Hybridoma-Zellen kloniert und diejenigen Zellklone, die die gewünschten monoklonalen Antikörper mit hoher Affinität zu Human-Renin produzieren, selektioniert.

Bevorzugt ist eine Immunisierung mit Human-Renin hoher Reinheit. Solches Human-Renin kann beispielsweise in an sich bekannter Weise aus Extrakten menschlicher Nieren mit Hilfe der Immuno-Affinitäts-Chromatographie gewonnen werden.

Bevorzugte Säugetiere zu Immunisierung sind Mäuse, insbesondere Balb/c-Mäuse, jedoch können auch andere Mäusestämme verwendet werden. Die Immunisierungen werden in an sich bekannter Weise vorgenommen, beispielsweise indem man drei bis sechs Injektionen parenteral, z.B. intraperitoneal, intravenös und/oder subcutan mit je zwischen 1 µg bis 20 µg gereinigtem Human-Renin in Abständen von ein bis sechs Wochen verabreicht, bevorzugt zusammen mit einem die Lymphozyten-Produktion anregenden Hilfsstoff, beispielsweise komplettem oder inkomplettem Freund, schen Adjuvans.

Antikörper-produzierende Zellen der immunisierten Tiere, vorzugsweise Milzzellen, werden zwei bis sechs Tage nach der letzten ("booster") Immunisierung den Tieren entnommen und mit Myeloma-Zellen einer geeigneten Zellinie in Gegenwart eines Fusions-Promotors fusioniert. Mehrere verschiedene Myeloma-Zellinien und daraus abgeleitete Zellinien sind bekannt als geeignete Fusionspartner. Bevorzugt sind Myeloma-Zellen, denen das Enzym Hypoxanthin-Guanin-Phosphoribosyl-Transferase (HGPRT) oder das Enzym Thymidin-Kinase (TK) fehlt und die deshalb in einem selektiven Kulturmedium, das Hypoxanthin, Aminopterin und Thymidin enthält (HAT-Medium), nicht überleben. Besonders bevorzugt sind Myeloma-Zellen und daraus hergestellte Zellinien, die in HAT-Medium nicht überleben und keine Immunglobuline oder Teile davon ausscheiden, beispielsweise die Zellinien X63—Ag8.653 und Sp2/0-Ag14. Als Fusions-Promoter kommen Sendai-Virus oder andere Paramyxoviren, gegebenenfalls in UV-inaktivierter Form, Kalzium-Ionen, oberflächenaktive Lipide, wie Lysolezithin, oder Polyäthylenglykol in Frage. Bevorzugt werden Myeloma-Zellen mit einem drei- bis zwanzigfachen Ueberschuss an Milzzellen

5

aus immunisierten Tieren in einer Lösung enthaltend 30 bis 50% Polyäthylenglykol eines Molekulargewichts zwischen 1000 und 4000 fusioniert.

Nach der Fusion werden die Zellen aufgeteilt und in selektivem HAT-Medium kultiviert, wobei nur Hybridoma-Zellen überleben, weil diese von den Myeloma-Zellen die Fähigkeit zum Wachstum in vitro und von Antikörper-produzierenden Zellen der immunisierten Tiere die fehlenden HGPRT- oder TK-Gene und damit die Fähigkeit zum Ueberleben in HAT-Medium vereinen.

Geeignete Kulturmedien für die Expansion der Hybridoma-Zellen sind die üblichen Standard-Kulturmedien, beispielsweise Dulbecco's Modified Eagle-Medium oder RPMI 1640-Medium, ergänzt mit 10—15% fötalem Kälber-Serum. Vorzugsweise weden zu Beginn des Zellwachstums sogenannte Feederzellen zugegeben, beispielsweise normale peritoneale Mäuse-Exsudatzellen, Milzzellen, Knochenmark-Makrophagen oder dergleichen. In regelmässigen Intervallen werden besagte Kulturmedien durch selektives HAT-Medium ergänzt, um ein Ueberwachsen der Hybridoma-Zellen durch gewöhnliche Myeloma-Zellen zu verhindern.

Die Zellkultur-Ueberstände der Hybridoma-Zellen werden daraufhin untersucht, ob sie die gewünschten monoklonalen Antikörper enthalten. Mit vorteil werden die Zellüberstände nicht nur in einem Immunoassay geprüft, der die Bindung von monoklonalen Antikörpern an Oberflächen-fixiertes Human-Renin zeigt, sondern gleichzeitig auch die Bindung an gelöstes Human-Renin und die Hemmung der Enzym-Aktivität von Human-Renin bestimmt. Ueberraschenderweise wurde nämlich festgestellt, dass monoklonale Antikörper, die an Oberflächen-fixiertes Renin gut binden, die Enzym-Aktivität von Renin nur wenig hemmen. Durch die Kombination verschiedenartiger Test-methoden gelingt es, Hybridoma-Zellen zu identifizieren, die monoklonale Antikörper ausscheiden, welche sowohl eine hohe Bindungsaffinität als auch eine starke Enzym-hemmende Wirkung gegenüber Human-Renin aufweisen. Derartige Hybridoma-Zellen werden durch begrenzende Verdünnung in an sich bekannter Weise kloniert, um ihre Einheitlichkeit sicherzustellen.

Die Erfindung betrifft im weiteren die Verwendung der monoklonalen Antikörper und ihrer Derivate mit hoher Affinität gegenüber Human-Renin zur qualitativen und quantitativen Bestimmung von Human-Renin und strukturell ähnlichem Renin, insbesondere in biologischen Flüssigkeiten. Beispielsweise können die erfindungsgemässen monoklonalen Antikörper und Antikörper-Derivate in irgendeinem der an sich bekannten Immunoassays verwendet werden, die die bindenden Wechselwirkungen zwischen Antigen (Human-Renin) und monoklonalem Antikörper nutzen. Beispiele für solche Assays sind Radioimmunoassays (RIA), Enzym-Immunoassays, Immuno-Fluoreszenz-Tests, Latex-Agglutination oder Hämagglutination.

Die erfindungsgemässen monoklonalen Antikörper können als solche oder als radioaktiv markierte Derivate einzeln oder in Kombination in einem Radioimmunoassay (RIA) eingesetzt werden. Irgendeine der bekannten Abwandlungen eines RIA kann verwendet werden, beispielsweise RIA in homogener Phase, Festphasen- oder heterogener RIA, einfacher RIA oder doppelter (Sandwich) RIA mit direkter oder indirekter (kompetitiver) Bestimmung von Human-Renin. Bevorzugt ist ein einfacher RIA, bei dem ein geeigneter Träger, beispielsweise die Plastik-Oberfläche einer Titerplatte oder eines Test-Röhrchens, z.B. aus Polystyrol, Polypropylen oder Polyvinylchlorid, Glas- oder Plastik-Perlen, Filterpapier, Dextran-, Celluloseacetat- oder Nitrocellulose-Blätter oder dergleichen mit einem oder einem Gemisch zweier, verschiedene Epitope erkennender monoklonaler Antikörper durch einfache Adsorption oder gegebenenfalls nach Aktivierung des Trägers beispielsweise mit Glutaraldehyd oder Bromcyan beschichtet, mit der Probelösung und einer Lösung einer bekannten Menge mit $^{125}$I radioaktiv markierten Human-Renins inkubiert und die auf besagtem Träger fixierte Radioaktivität bestimmt wird.

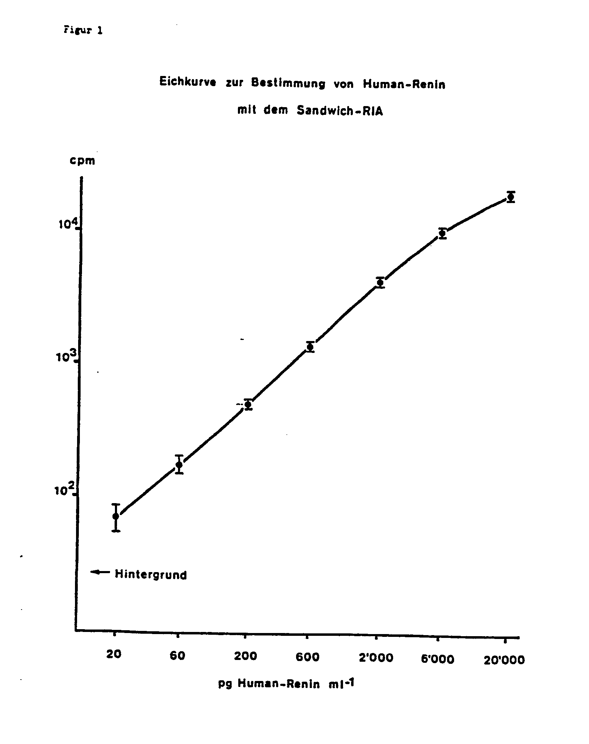

Besonders bevorzugt ist ein Sandwich-Radioimmunoassay, bei dem ein mit einem oder zwei monoklonalen Antikörpern beschichteter Träger mit der Probelösung und einer Lösung eines mit $^{125}$I radioaktiv markierten monoklonalen Antikörpers inkubiert wird, wobei der gelöste monoklonale Antikörper ein anderes Epitop von Human-Renin erkennt als der oder die Träger-gebundenen monoklonalen Antikörper, und die Menge Renin in der Probelösung durch Messung der an den Träger gebundenen Radioaktivität bestimmt wird. In Figur 1 ist die gemessene, auf den Träger gebundene Radioaktivität in Abhängigkeit der in der Probelösung enthaltenen Menge Human-Renin dargestellt, wie sie mit dem in Beispiel 8.4 näher beschriebenen Sandwich-RIA gemessen werden kann. Die Empfindlichkeit des Testes erlaubt die zuverlässige, quantitative Bestimmung von nur 1 pg bis zu mehr als 100 pg Human-Renin in 50 µl Probelösung in weniger als 4 Stunden. Die Verwendung der erfindungsgemässen monoklonalen Antikörper mit hoher Affinität für Renin ermöglicht somit die quantitative Bestimmung von Human-Renin bei Konzentrationen, wie sie in biologischen Flüssigkeiten und insbesondere in Blut normalerweise auftreten, und erlaubt damit eine schnelle und zuverlässige Diagnose von Reninbedingtem Bluthochdruck.

Die erfindungsgemässen monoklonalen Antikörper können als solche oder als Enzym-konjugierte Derivate einzeln oder in Kombination in einem Enzym-Immunoassay eingesetzt werden. Solche Immunoassays umfassen Test-Anordnungen, bei denen Enzym-markierte erfindungsgemässe monoklonale Antikörper-Derivate, an sich bekannte Enzym-markierte Antikörper, die Epitope der erfindungsgemässen oder anderer anti-Human-Renin-Antikörper erkennen und binden, oder Enzymmarkiertes Human-Renin eingesetzt werden.

Bevorzugt ist ein ELISA (enzyme-linked immunosorbent assay), in dem ein Träger, wie er oben für einen einfachen RIA-Test beschrieben ist, mit einem oder zwei erfindungsgemässen monoklonalen

6

Antikörpern beschichtet, mit einer Human-Renin enthaltenden Probelösung und dann einem polyklonalen Serum gegen Human-Renin, beispielsweise Kaninchen-Serum gegen Human-Renin, inkubiert, schliesslich die gebundenen Antikörper des polyklonalen Serums durch Enzym-markiert Antikörper, die diese erkennen und binden, entwickelt und durch Enzym-Substrat-Reaktion die gebundene Menge Human-Renin bestimmt wird. Ein solcher Enzym-markierter Antikörper ist beispielsweise ein Phosphatasemarkiertes Ziegen-anti-Kaninchen-Immunglobulin.

Ganz besonders bevorzugt ist ein ELISA, bei dem ein mit einem oder zwei erfindungsgemässen monoklonalen Antikörpern beschichteter Träger mit einer Human-Renin enthaltenden Probelösung und einer Lösung eines mit einem Enzym konjugierten erfindungsgemässen monoklonalen Antikörpers inkubiert wird, wobei der gelöste monoklonale Antikörper ein anderes Epitop von Human-Renin erkennt als der oder die Träger-gebundenen monoklonalen Antikörper. Durch eine Enzym-Substrat-Reaktion, die beispielsweise zu einer Farbänderung führt und von Auge oder mit optischen Messgeräten verfolgt werden kann, wird die Menge des gebundenen Enzyms bestimmt, die proportional zur Menge Human-Renin in der Probelösung ist.

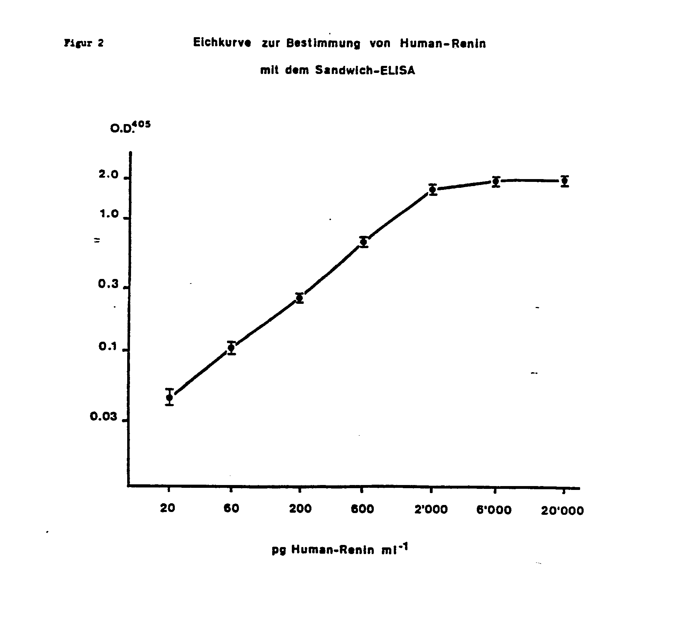

In Figur 2 ist die gemessene optische Dichte bei der maximalen Extinktion des freigesetzten farbigen Enzymsubstrats in Abhängigkeit von der in der Probelösung enthaltenen Menge Human-Renin dargestellt, wie sie mit dem in Beispiel 9.3 näher beschriebenen ELISA gemessen werden kann. Die Empfindlichkeit des ELISA erlaubt die zuverlässige, quantitative Bestimmung von weniger als 1 pg bis zu 100 pg Human-Renin in 50 µl Probelösung in weniger als 5 Stunden. Gegenüber dem oben beschriebenen bevorzugten Sandwich-RIA weist der ELISA bei gleicher oder leicht höherer Empfindlichkeit den Vorteil auf, dass keine komplizierten Messgeräte wie zur Bestimmung der Radioaktivität nötig sind und weniger hohe Sicherheitsanforderungen wie beim Umgang mit radioaktiven Substanzen erfüllt werden müssen.

Bevorzugte Enzyme in den erfindungsgemässen Enzym-Immunoassays sind Meerrettich-Peroxidase, die beispielsweise mit den Enzym-Substraten 5-Aminosalicylsäure, o-Phenylendiamin, 3,3'-Dimethoxybenzidin, 2,2'-Azino-bis-(3-äthylbenzthiazolin-6-sulfonsäure) oder dergleichen entwickelt werden kann, und insbesondere alkalische Phosphatase, die beispielsweise aus dem Enzym-Substrat p-Nitrophenylphosphat p-Nitrophenol freisetzt.

Die erfindungsgemässe Verwendung von monoklonalen Antikörpern mit hoher Affinität gegenüber Human-Renin zur qualitativen und quantitativen Bestimmung von Human-Renin umfasst auch andere an sich bekannte Immunoassays, beispielsweise Immunofluoreszenz-Tests unter Verwendung von Antikörper- oder Antigen-Konjugaten mit fluoreszierenden Stoffen, Latex-Agglutination mit Antikörper- oder Antigenbeschichteten Latex-Partikeln oder Hämagglutination mit Antikörper- oder Antigenbeschichteten roten Blutkörperchen oder dergleichen.

Die beschriebenen Immunoassays können angewendet werden zur Bestimmung der in bioligischen Flüssigkeiten, insbesondere in menschlichem Blut vorhandenen Menge Human-Renin und damit zur Diagnose von Renin-bedingtem Bluthochdruck. Dabei wird nicht nur jene Menge Human-Renin gemessen, die als aktives Enzym Angiotensinogen (Renin-Substrat) in Angiotensin I umwandelt, sondern auch alle andern Formen von Human-Renin, die die gleichen, vom Antikörper erkannten antigenen Determinanten aufweisen. Insbesondere wird mit den beschriebenen Immunoassays die totale Menge Human-Renin bestimmt, die aktives Enzym und inaktives Human-Renin bzw. Human-Pro-Renin umfasst. Die Immunoassays sind auch anwendbar auf die Bestimmung von Primaten-Renin, insbesondere Renin von Marmosets (Pinselohräffchen, Callitrix jacchus), oder anderem tierischem Renin, das ähnliche oder identische antigene Determinanten aufweist.

Die Erfindung betrifft auch Test-Kits zur Bestimmung von Human-Renin und strukturell ähnlichem Renin enthaltend monoklonale Antikörper mit hoher Affinität zu Human-Renin und/oder Derivate davon und gegebenenfalls Hilfsmittel.

Erfindungsgemässe Test-Kits für einen Radioimmunoassay enthalten beispielsweise einen geeigneten Träger, gegebenenfalls gefriergetrocknete oder konzentrierte Lösungen eines oder mehrerer erfindungsgemässer monoklonaler Antikörper, Lösungen eines radioaktiv markierten erfindungsgemässen monoklonalen Antikörpers oder von radioaktiv markiertem Human-Renin, Standardlösungen von Human-Renin, Puffer-Lösungen und gegebenenfalls Detergentien zur Verhinderung von umspezifischer Adsorption und Aggregatbildung, Pipetten, Reaktionsgefässe, Eichkurven und dergleichen.

Erfindungsgemässe Test-Kits für einen Enzym-Immunoassay enthalten beispielsweise einen geeigneten Träger, gegebenenfalls gefriergetrocknete oder konzentrierte Lösungen eines oder mehrerer efindungsgemässer monoklonaler Antikörper, gegebenenfalls gefriergetrocknete oder konzentrierte Lösungen eines Enzym-markierten erfindungsgemässen monoklonalen Antikörpers, von Enzymmarkiertem Human-Renin, eines polyklonalen anti-Human-Renin-Serums und/oder von Enzym-markierten monoklonalen oder polyklonalen Antikörpern, die die erfindungsgemässen oder andere anti-Human-Renin-Antikörper erkennen und binden, Enzym-Substrate in fester oder gelöster Form, Standardlösungen von Human-Renin, Puffer-Lösungen, Detergentien, Pipetten, Reaktionsgefässe, Eichkurven, Farbskala-Tafeln und dergleichen.

Die Erfindung betrifft in weiteren die Verwendung der monoklonalen Antikörper und ihrer Derivate mit hoher Affinität zu Human-Renin zur Reinigung von Human-Renin und strukturell ähnlichem Renin. Beispielsweise kann Human-Renin oder strukturell ähnliches Renin mit an sich bekannten Trennmethoden, deren Trennwirkung auf bindenden Wechselwirkungen zwischen monoklonalen Antikörper und antigenen Determinanten von Renin beruhen, gereinigt werden. Eine bevorzugte Trennmethode ist die Immuno-Affinitäts-Chromatographie. Ein geeignetes Trägermaterial auf anorganischer oder organischer Basis, beispielsweise vernetzte Agarose, Dextran oder Polyacrylamid in geeignet funktionalisierter Form, wird auf an sich bekannte Weise, gegebenenfalls unter vorheriger Aktivierung, mit den erfindungsgemässen monoklonalen Antikörpern oder Antikörper-Derivaten belegt. Dazu wird beispielsweise ein Trägermaterial enthaltend aktivierte Esterfunktionen in einer wässrigen Puffer-Lösung suspendiert, mit einer Lösung des monoklonalen Antikörpers vermischt, anschliessend ungebundene monoklonale Antikörper ausgewaschen und unbesetzte reaktive Stellen des Trägermaterials blockiert.

Die Erfindung betrifft auch pharmazeutisch Präparate enthaltend einen monoklonalen Antikörper mit hoher Affinität zu Human-Renin oder ein Derivat davon und gegebenenfalls pharmazeutische Hilfsmittel. Geeignete Derivate sind Bruchstücke von monoklonalen Antikörpern, beispielsweise Fab, Fab' oder F(ab')$_2$-Fragmente, die ihre Spezifität für die antigenen Determinanten von Human-Renin beibehalten haben. Die pharmazeutischen Zubereitungen enthalten beispielsweise monoklonale Antikörper oder Bruchstücke davon in einer wirksamen Menge zusammen oder im Gemisch mit anorganischen oder organischen, festen oder flüssigen pharmazeutisch verwendbaren Trägerstoffen.

Bevorzugt sind pharmazeutische Präparate für parenterale, beispielsweise intramuskuläre oder insbesondere intravenöse Anwendung. Solche Zubereitungen sind isotonische wässrige Lösungen oder Suspensionen, die gewünschtenfalls erst kurz vor Gebrauch aus lyophilisierten Präparaten oder konzentrierten Lösungen hergestellt werden. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natrium-Carboxymethylcellulose, Dextran, Polyvinylpyrrolidon oder Gelatine, enthalten.

Die Herstellung der Injektionspräparate erfolgt in üblicher Weise unter antimikrobiellen Bedingungen, ebenso das Abfüllen in Ampullen oder Vials sowie das Verschliessen der Behälter.

Die Erfindung betrifft im weiteren die Verwendung der monoklonalen Antikörper mit hoher Affinität zu Human-Renin oder Derivate davon zur Behandlung von hohem Blutdruck und Herzinsuffizienz.

Für den Nachweis der blutdrucksenkenden Wirkung der erfindungsgemässen monoklonalen Antikörper wird ein in vivo Test an Marmosets (Pinselohräffchen, Callitrix jacchus) verwendet. Die erfindungsgemässen monoklonalen Antikörper hemmen Marmoset-Renin in etwa gleichem Masse wie Human-Renin, erkennen und binden also antigene Determinanten, die in Marmoset- und Human-Renin ähnlich oder identisch sind.

In einer üblichen Versuchsandordnung werden normotensive Marmosets (Callitrix jacchus) beider Geschlechter mit einem Körpergewicht von etwa 300 g mit einer normalen Salz-Diät (NAFAG, Na$^+$: 100 mmol/kg, K$^+$: 250 mmol/kg) ergänzt durch Früchte gefüttert. Die endogene Freisetzung von Renin wird durch eine intravenöse Injektion von Furosemid (5 mg/kg) angeregt. 45 Min. später werden die monoklonalen Antikörper über ein Katheter in die laterale Schwanzvene injiziert und ihre Wirkung auf den Blutdruck und die Herzfrequenz über ein Katheter in der Oberschenkelarterie gemessen. 2 Std. nach der Injektion der monoklonalen Antikörper wird Teprotid, das das Angiotensin-umwandelnde Enzym (ACE) hemmt, injiziert (1 mg/kg i.v.) und seine Wirkung auf den Blutdruck verfolgt. In Kontrollexperimenten werden anstelle der erfindungsgemässen monoklonalen anti-Human-Renin-Antikörper physioligische Kochsalzlösung (1 ml/kg) oder der unspezifische monoklonale Myeloma-Antikörper MOPC 21 (0,1 mg/kg) injiziert.

Unter diesen Versuchsbedingungen bewirkt eine einmalige Injektion des monoklonalen Antikörpers R 3—36—16 in einer Dosis von 0,01 mg/kg i.v. eine Blutdrucksenkung um 18±5 mm Hg (n = 4) bis zur Dauer von 2 Std. ohne Veränderung der Herzschlagrate. Während 30 Min. wird die Plasma-Renin-Aktivität vollständig gehemmt. Die Injektion von Teprotid nach einer Antikörpergabe bewirkt keine zusätzliche Blutdrucksenkung. Höhere Dosen an monoklonalem Antikörper R 3—36—16 (0,1 mg/kg i.v.) haben eine ähnliche blutdrucksenkende Wirkung ohne Beeinflussung der Herzschlagrate, niederigere Dosen (0,001 mg/kg i.v.) bleiben ohne Auswirkungen.

Die nachfolgenden Beispiele illustrieren die oben beschriebene Erfindung, schränken jedoch deren Umfang in keiner Weise ein.

Die in den Beispielen verwendeten Abkürzungen haben folgende Bedeutung:

| | |
|---|---|
| BSA | Rinderserum-Albumin |
| ELISA | Enzym-Assay (enzyme-linked immunosorbent assay) |
| HAT | Hypoxanthin/Aminopterin/Thymidin |
| IC 50 | Konzentration, bei der 50% Hemmung beobachtet wird |
| PBS | Phosphat-gepufferte physioligische Kochsalzlösung |
| RIA | Radioimmunoassay |
| SDS | Natriumdodecylsulfat |
| Tris | Tris-(hydroxymethyl)-aminomethan |

Einheiten:

M        Mol/liter
GU       Goldblatt-Einheiten
cpm      Zahl pro Minute (radioaktiver Zerfall)

## Beispiel 1

Reinigung von Human-Renin

Human-Renin wird aus menschlichen Nieren gewonnen und in an sich bekannter Weise mit Hilfe der Immunabsorptions-Technik gereinigt.

1 kg mechanisch zerhackte Niere wird mit Wasser extrahiert und mit Schwefelsäure auf pH 2,8 angesäuert. Die NaCl-Konzentration wird auf 0,8 M eingestellt und das Renin mit Ammoniumsulfat (Endkonzentration 2,3 M) gefällt. Die Fällung wird gegen 0,1 M Phosphatpuffer (pH 7,4) dialysiert und weist eine Konzentration von 0,13 GU Renin pro mg Protein auf. Der Renin-Gehalt wird durch Bestimmung von Angiotensin I nach Inkubation der Probe mit einem Ueberschuss an Reninfreiem Plasma als Quelle von Angiotensinogen gemessen (NEN, New England Nuclear Angiotensin I Radioimmunoassay Kit) und in Goldblatt-Einheiten (Goldblatt units, GU) im Vergleich zu einem Renin-Standard (MRL, international reference standard 68/356 [5]) ausgedrückt. Die Proteinkonzentration wird durch Färbung mit Coomassie Blue und Vergleich mit Rinderserum-Albumin (bovine serum albumin, BSA) als Standard bestimmt.

Der dialysierte Nierenextrakt wird auf eine Immuno-Affinitätskolonne gegeben, die 38 mg monoklonalen anti-Human-Renin-Antikörper F 15 (Clin-Midy, Montpellier) [6] gebunden an 6 ml Affi-Gel® 10 (Biorad) enthält. Ungebundene Komponeten des Plasmas werden mit 0,1 M Phosphatpuffer weggewaschen und gebundenes Renin mit 0,1 M Citrat-Phosphat-Puffer (pH 4,5) eluiert. Das Eluat wird auf einer Amikon-PM-10®-Membran konzentriert, mit PBS (phosphate buffered saline) verdünnt, eingefroren und bei −80°C aufbewahrt. Auf diese Weise wird ca. 70% des im rohen Nierenextrakt vorhandenen Renins mit einer spezifischen Aktivität von 400 GU/mg gewonnen, was einer 3000-fachen Anreicherung entspricht. Die Reinheit der Präparation wird mit SDS-Polyacrylamid-Gel-Elektrophorese und Hochdruckflüssigkeits-Chromatographie analysiert und auf >80% geschätzt.

## Beispiel 2

Herstellung der Hybridoma-Zellen

2.1 Immunisierung von Mäusen mit Human-Renin

BALB/c-Mäuse werden mit dem gereinigtem Human-Renin aus Beispiel 1 folgendermassen immunisiert:

Maus 1 und 2 werden 10 GU, entsprechend ca. 10 µg Renin, in komplettem Freund'schen Adjuvans (CFA) verteilt auf zwei Fusspfoten und zwei subcutane (s.c.) Injektionen verabreicht. Am 28. Tag danach werden weitere 2 GU Renin und am 46. Tag 5 GU Renin in PBS intravenös (i.v.) injiziert. Die Milz wird am 50. Tag zur Fusion entnommen.

Maus 3 und 4 werden 20 GU Renin in CFA, verteilt wie für Maus 1 und 2, am 35. Tag 2 GU Renin in inkomplettem Freund'schen Adjuvans (IFA) s.c., am 60. Tag 2 GU Renin in PBS intraperitoneal (i.p.) und am 78. Tag 5 GU Renin in PBS i.v. verabreicht. Jener Maus mit dem höheren Serumtiter gegen *in vitro* Renin-Aktivität (Maus 3) wird am 82. Tag die Milz zur Fusion entnommen.

2.2 Zellfusion

Alle Fusionsexperimente werden unter Verwendung der Sp2/O-Ag14 Zell-Linie [7] im wesentlichen entsprechend dem Verfahren von Köhler und Milstein [1] durchgeführt. $10^8$ Milzzellen aus Beispiel 2.1 werden mit $10^7$ Myeloma-Zellen in Gegenwart von 1 ml 50%-igem Polyäthylenglykol (PEG 1500, Serva) vermischt. Nach dem Waschen werden die Zellen in 48 ml Standard Dulbecco's minimum essential Medium (Gibco Nr. 0422501) resuspendiert. 15% fötales Kälber-Serum und $3 \times 10^6$ normale peritoneale Mäuse-Exsudatzellen pro Fusion werden als Feederzellen zugegeben. Die Zellen werden in 48 × 1 ml-Costarplatten verteilt. Die Kulturen werden zweimal täglich mit Standard HAT-Selektivmedium [1] während 3 bis 6 Wochen gefüttert. Nach dem Wachstum der Hybrid-Zellen werden die Kultur-Ueberstände auf Bindung an Renin (Beispiel 4) und auf Hemmung der Renin-Aktivität (Beispiel 5) untersucht. Die Klonierung der Hybridoma-Zellen erfolgt durch begrenzende Verdünnung in Mikrotiterplatten. Alle Zellinien werden mindestens zweimal sequentiell kloniert.

Es werden Klone von zehn verschiedenen Mutterkulturen ausgewählt und expandiert. R1, R2 und R3 bezeichnen Hybridoma-Zellen und daraus gewonnene Antikörper aus der Fusion der Milzzellen von Maus 1, 2 bzw. 3. Die Klon-Nummer und eine allenfalls angegebene Subklon-Nummer werden nach Bindestrichen beigefügt (Tabelle 1).

## Beispiel 3

Isolierung und Reinigung von monoklonalen Antikörpern

8—10 Wochen alte BALB/c-Mäuse (Tierfarm Sisseln) werden i.p. mit 0,3 ml Pristan (Aldrich) vorbehandelt. 2—3 Wochen später werden jeweils $2-5 \times 10^6$ klonierte Hybridoma-Zellen und 0,2 ml Pristan i.p. injiziert. 8—10 Tage danach werden die Mäuse wiederholt punktiert, die so gewonnene Aszitesflüssigkeit bei 800 g zentrifugiert und der dabei erhaltene geklärte Ueberstand bei −20°C gesammelt.

Aufgetaute Asziteslösung wird 60 Min. bei 50 000 g zentrifugiert, oben aufschwimmendes Fett entfernt und eine Konzentration von 10—12 mg Protein pro ml eingestellt. Die Proteinkonzentration wird durch Messung der optischen Dichte bei 280 nm (OO$_{280}$) bestimmt. Als Standard dient eine 1%ige Lösung (G/V) von murinem Immunglobulin mit OO$_{280}$ = 12 (1 cm Schichtdicke). Durch langsames Zutropfen von 0,9 Volumenäquivalenten gesättigter Ammoniumsulfatslösung bei 0°C unter Rühren wird eine rohe Immunglobulin-Fraktion gefällt, welche in 20 mM Tris-HCl/50 mM NaCl (pH 7,9) gelöst und dialysiert wird. Anschliessend wird mit 20 mM Tris-HCl (pH 7,9) zweifach verdünnt und auf eine DEAE—D52 Cellulose-Säule (Whatman) gegeben. Die Immunglobulin G-Fraktion wird mit einem Puffer von 20 mM Tris-HCl/80 mM NaCl (pH 7,9) aus der Säule eluiert und nach erneuter Ammoniumsulfat-Fällung (siehe oben) mit PBS auf eine Proteinkonzentration von 10 mg/ml eingestellt. Die Reinheit der monoklonalen Antikörper-Präparationen wird mit SDS-Polyacrylamid-Gel-Elektrophorese überprüft und ist besser als 95%.

### Beispiel 4
Bindung der monoklonalen Antikörper an Human-Renin
4.1 Enzymimmuno-Assay (ELISA)

Die Reaktivität gegen Oberflächen-gebundenes Renin wird in einem an sich bekannten ELISA-Verfahren [8] sowohl zur Selektion der gewünschten monoklonale Antikörper-produzierenden Hybridoma-Zellen nach Zellhybridisierung (Beispiel 2.2) als auch zur quantitativen Bestimmung der Bindung an Renin bei gereinigten monoklonalen Antikörpern verwendet. 100 ng gereinigtes Renin (Beispiel 1) in 50 µl 0,05 M Natriumbicarbonat-Puffer (pH 9,6) werden während 2 Std. bei 37°C und 15 Std. bei 4°C in Plastik-Mikrotiterplatten inkubiert. Nach Waschen mit PBS werden noch vorhandene Protein-reaktive Stellen auf der Plastikoberfläche durch Inkubation während 2 Std. bei 37°C mit 150 µl PBS-Tween®-Puffer (0,05% Tween® 20 in PBS enthaltend 0,2% NaN$_3$, pH 7,4) gesättigt und die Platte mit PBS gewaschen. 100 µl der auf monoklonale anti-Renin-Antikörper zu messenden Lösung (Zellkulturüberstände oder gereinigte monoklonale Antikörperlösungen) und entsprechende Verdünnungen davon werden während 2 Std. bei 37°C inkubiert und nach Waschen der Platte die gebundenen monoklonalen Maus-Antikörper durch Inkubation mit 100 µl einer entsprechend vorbestimmten Verdünnung einer Präparation von Phosphatase-markiertem Kaninchen IgG anti-Maus-Immunglobulin entwickelt. Die aufgenommene Menge Enzym wird durch Inkubation (30 Min., 37°C) mit 100 µl Lösung des Enzym-Substrates p-Nitrophenylphosphat (1 mg/ml in 10% Diäthanolamin-Puffer enthaltend 0,5 mM MgCl$_2$, pH 9,8) und Messen der optischen Dichte des umgesetzen Produktes bei 405 nm (OD$_{405}$) mit einem Multiscan Photometer (Flow Irvine, Schottland) bestimmt. Als Kontrolle werden anstelle der monoklonale anti-Renin-Antikörper enthaltenden Lösungen Präparationen des unspezifischen monoklonalen Myeloma-Antikörpers MOPC 21 (Bionetics Labs., Kensington, USA) und eines monoklonalen Hybridoma-Antikörpers spezifisch für Phosphorylcholin verwendet.

Die Konzentration an gereinigtem monoklonalem Antikörper, welche in diesem Assay mit Oberflächen-Gebundenem Human-Renin schliesslich eine OD$_{405}$ von 0,1 ergibt, ist in Tabelle 1 aufgeführt. Die gefundenen Konzentrationen liegen im Bereich von $5,8 \times 10^{-7}$ bis $7,5 \times 10^{-11}$ M, wobei Konzentrationen grösser als $10^{-7}$ M keine spezifische Bindung darstellen, weil auch die monoklonalen Kontroll-Antikörper im Assay Werte dieser Grössenordnung ergeben. Besonders effiziente Bindung an fixiertes Renin (ELISA) werden bei den monoklonalen Antikörpern R 3—17—7 und R3—17—8 gefunden, aber auch die monoklonalen Antikörper R 3—48, R 3—21 und R 3—27 binden bei Konzentrationen unter $5 \times 10^{-10}$ M.

4.2 Radioimmunoassay (RIA) mit $^{125}$I-markiertem Renin

Zur Bestimmung der Reaktivität gegenüber löslichem Renin werden verschiedene Verdünnungen einer monoklonalen Antikörper-Probe in 50 µl RIA-Puffer (1% BSA in PBS, 0,2% NaN$_3$, pH 7,4) mit 50 µl $^{125}$I-markiertem Renin (80000 cpm entsprechend 2,5 ng Protein, Beispiel 8.1) in demselben Puffer versetzt und in Polyvinylchlorid-Mikrotiterplatten während 2 Std. bei 37°C und 15 Std. bei 4°C inkubiert. Die Antikörper-gebundene Menge $^{125}$I-Renin wird durch Fällung der monoklonalen Antikörper als Immunkomplexe erhalten, indem 50 µl einer entsprechenden Verdünnung eines Kaninchen-anti-Maus-Immunglobulin-Serums in 3% Polyäthylenglykol 6000 zugesetzt und während 30 Min. bei 37°C und 30 Min. bei 4°C inkubiert wird. Die Fällung wird durch Aufschlämmen und Zentrifugieren mehrmals gewaschen und die Radioaktivität nach Schneiden der Titerplatte im Gammazähler bestimmt.

Die Konzentration an gereinigtem monoklonalem Antikörper, welche noch 50% der maximal präzipitierbaren Radioaktivität aus der $^{125}$I-Renin-haltigen Lösung ausfällt (IC 50), ist ein Mass für die Bindung an gelöstes Renin und in Tabelle 1 angegeben. Besonders stark binden die monoklonalen Antikörper R 3—36—16 und R 3—47 gelöstes Renin, obwohl sie im ELISA (Beispiel 4.1) nur eine geringe Affinität zu Oberflächen-gebundenem Renin zeigen.

### Beispiel 5
Hemmung der Renin-Aktivität in vitro
5.1 Bestimmung der Hemmung der Human-Renin-Aktivität

Renin-depletiertes normales menschliches Plasma enthaltend Angio-tensinogen wird mit gereinigtem

<div style="text-align:center">10</div>

Human-Renin (Beispiel 1) auf 250 pg/ml Renin ergänzt. Je 50 µl dieses vorbehandelten Plasmas werden mit verschiedenen Verdünnungen der zu analysierenden monoklonalen Antikörper-Proben in 50 µl 0,1 M Tris-Acetat-Puffer (pH 7,4) in Gegenwart von 2,3-Dimercapto-1-propanol und 8-Hydroxychinolin, die die Umsetzung von Angiotensin I in Angiotensin II blockieren, 2 Std. bei 4°C und 1 Std. bei 37°C inkubiert. Die enzymatische Reaktion wird durch Abkühlung auf 4°C gestoppt..Die gebildete Menge von Angiotensin I wird mit einem RIA (NEN, New England Nuclear Angiotensin I Radioimmunoassay Kit) bestimmt. Zu diesem Zweck werden die Proben mit [125]I-markiertem Angiotensin I-Tracer und einer entsprechenden Menge an Antiserum gegen Angiotensin I versehen, nach Inkubation während 15 Std. bei 4°C ungebundenes Angiotensin I an Aktivkohle adsorbiert und das Antikörper-gebundene [125]I-markierte Angiotensin I im Ueberstand mit einem Gammazähler bestimmt. In diesem System ergibt die Positivkontrolle (Proben ohne monoklonale Antikörper) 10 ng Angiotensin I pro ml und Std.

Die Konzentration an gereinigtem monoklonalem Antikörper, bei der 50% der maximal erreichbaren Hemmung der Human-Renin-Aktivität (IC 50) beobachtet wird, ist in Tabelle 1 aufgeführt und erstreckt sich von $>10^{-6}$ M bis $1,3 \times 10^{-11}$ M. Die monoklonalen Antikörper R 3—17—7 und R 3—48 hemmen die Renin-Aktivität nicht signifikant, obwohl sie Renin im ELISA (Beispiel 4.1) stark binden. Signifikante, jedoch schwache Hemmung der Renin-Aktivität zeigen die monoklonalen Antikörper R 1—19, R 1—20, R 3—17—8, R 2—12 und R 3—21. Ausgeprägte Hemmung der Renin-Aktivität wird bei den monoklonalen Antikörpern R 3—27, R 2—1 und besonders R 3—47 und R 3—36—16 beobachtet.

Die monoklonalen Antikörper R 3—21 und R 2—1 hemmen Renin nur bis zu 60% über den gesamten untersuchten Konzentrationsbereich (bis $10^{-6}$M), R 3—27 zeigt eine maximale Hemmung von nur 35%. Diese monoklonalen Antikörper erkennen und binden antigene Determinanten (Epitope), die sehr wahrscheinlich in der Nähe des aktiven Zentrums von Renin liegen. Mi ihrer Bindung beeinträchtigen sie die Enzymaktivität durch sterische Beeinflussung oder begünstigen eine allosterische Konfiguration des Reninmoleküls, welche noch eine kleinere Substratwechselzahl zulässt. Die nur schwach hemmenden monoklonalen Antikörper R 1—19 und R 1—20 hingegen verursachen bei hohen Konzentrationen vollständige Reninhemmung genau wie die monoklonalen Antikörper mit der grössten Affinität, die Antikörper R 3—47 und R 3—36—16.

Tabelle 1: Reaktivität gegenüber Human-Renin [a]

| Gruppe | Klon-Bezeichnung | Antikörper-Klasse | Bindung an Renin | | Hemmung der Renin-Aktivität (Beispiel 5.1) | |
|---|---|---|---|---|---|---|
| | | | ELISA (Bsp. 4.1) | RIA (Bsp. 4.2) | IC 50 | max.erreichbare Hemmung (%) |
| 1 | R 3-17-7 | γ1 | $9,5 \times 10^{-11}$ | b) | $> 10^{-6}$ | b) |
| | R 2-12 | γ2 | $4,2 \times 10^{-9}$ | $1,5 \times 10^{-7}$ | $> 10^{-6}$ | b) |
| | R 3-17-8 | γ1 | $7,5 \times 10^{-11}$ | $4,9 \times 10^{-9}$ | $8,0 \times 10^{-8}$ | b) |
| | R 3-48 | γ1 | $1,1 \times 10^{-10}$ | $2,1 \times 10^{-8}$ | $8,5 \times 10^{-8}$ | b) |
| 2 | R 3-21 | γ1 | $1,5 \times 10^{-10}$ | $9,1 \times 10^{-10}$ | $1,4 \times 10^{-9}$ | 60 |
| | R 3-27 | γ1 | $4,8 \times 10^{-10}$ | $5,7 \times 10^{-10}$ | $1,0 \times 10^{-10}$ | 35 |
| | R 2-1 | γ1 | $5,8 \times 10^{-7}$ | $4,1 \times 10^{-9}$ | $5,7 \times 10^{-10}$ | 60 |
| 3 | R 3-36-16 | γ1 | $6,5 \times 10^{-8}$ | $1,7 \times 10^{-10}$ | $1,3 \times 10^{-11}$ | 100 |
| | R 3-47 | γ1 | $8,2 \times 10^{-9}$ | $2,4 \times 10^{-10}$ | $2,0 \times 10^{-11}$ | 100 |
| 4 | R 1-19 | γ1 | $9,3 \times 10^{-8}$ | $2,5 \times 10^{-8}$ | $1,4 \times 10^{-8}$ | 100 |
| | R 1-20 | γ1 | $3,7 \times 10^{-7}$ | $2,0 \times 10^{-8}$ | $1,4 \times 10^{-8}$ | 100 |

a) Konzentrationen in Mol pro Liter (M)
b) nicht bestimmt

EP 0 158 599 B1

5.2 Vergleich der Bindung an Human-Renin und der Hemmung der Human-Renin-Aktivität

Die monoklonalen Antikörper können bezüglich ihrer Bindungs- und Hemmeigenschaften in vier verschiedene Gruppen eingeteilt werden. Prinzipiell sind monoklonale Antikörper zu erwarten, welche Renin nur binden und solche, welche Renin binden und gleichzeitig dessen enzymatische Aktivität beeinträchtigen. Gruppe 1 (Tabelle 1) fasst jene monoklonalen Antikörper zusammen, die Renin binden, jedoch dessen katalytische Aktivität nicht oder nur ganz schwach hemmen. Diese monoklonalen Antikörper zeigen zudem eine mindestens 20-fach höhere Affinität gegenüber Oberflächen-gebundenem Renin (ELISA) im Vergleich zur Affinität gegenüber Renin in Lösung (RIA). Die monoklonalen Antikörper aller anderen Gruppen weisen keine Präferenz zu Oberflächen-gebundenem Renin auf. Monoklonale Antikörper der Gruppe 2 hemmen die Renin-Aktivität schon bei geringen Konzentrationen, jedoch nur partiell, d.h. nur zu einem gewissen, für den monoklonalen Antikörper charakteristischen Prozentsatz. Gruppe 3 umfasst monoklonale Antikörper, welche die Renin-Aktivität schon bei extrem niedrigen Konzentrationen vollständig hemmen. Gleichzeitig binden sie gelöstes Renin bei geringen Konzentrationen von 1 bis $3 \times 10^{-10}$. Interessanterweise reagieren diese monoklonalen Antikörper kaum mit Oberflächen-gebundenem Renin. Solche monoklonale Antikörper würden demzufolge in einem üblichen Primärscreen basierend auf der ELISA-Methode nicht erfasst. Gruppe 4 umfasst monoklonale Antikörper, welche die Renin-Aktivität vollständig hemmen, aber ca. 1000-mal schwächer sind als monoklonale Antikörper der Gruppe 3. Sie binden zudem Oberflächen-gebundenes Renin (ELISA) und lösliches Renin (RIA) etwa gleich gut. Die Bindung im ELISA (bei ca. $10^{-7}$ M) ist allerdings nicht signifikant verschieden von derjenigen unspezifischer monoklonaler Kontroll-Antikörper.

5.3 Bestimmung der Hemmung von Ratten- und Marmoset-Renin-Aktivität

Analog der in Beispiel 5.1 beschriebenen Weise werden die zu analysierenden monoklonalen Antikörper-Proben auf Hemmung der Aktivität von Ratten-Renin und von Marmoset-Renin geprüft. Dazu wird jedoch unbehandeltes, d.h. normale Mengen Angiotensinogen und Renin enthaltendes Ratten- bzw. Marmoset-Plasma anstelle des vorbehandelten Human-Plasmas eingesetzt.

Keiner der in Tabelle 1 aufgeführten monoklonalen Antikörper hemmt Ratten-Renin.

Die monoklonalen Antikörper R 3—27, R 3—36—16, R 3—47 und R 1—20 hemmen Marmoset-Renin im gleichen Masse wie Human-Resin (gleiche IC 50-Werte). Die von entsprechenden monoklonalen Antikörper erkannte Determinante ist deshalb in Human-Renin und Marmoset-Renin identisch oder zumindest sehr ähnlich. Da die monoklonalen Antikörper R 3—36—16 und R 3—47 Human-Renin sehr effizient hemmen (Tabelle 1), ist eine Homologie der katalytischen Stelle der Enzyme Human-Renin und Marmoset-Renin gezeigt.

Der monoklonale Antikörper R 1—19 hemmt die Aktivität von Marmoset-Renin bei dreimal geringerer Konzentration (IC 50) als diejenige von Human-Renin. Dieser monoklonale Antikörper reagiert also besser mit einem Molekül, das nicht zur Immunisierung (Beispiel 2.1) verwendet worden ist.

Der monoklonale Antikörper R 2—1 hemmt die Aktivität von Marmoset-Renin erste bei fünfzehnmal höherer Konzentration (IC 50) als diejenige von Human-Renin. Dieser monoklonale Antikörper erkennt deshalb eine Determinante, die in Marmoset- und Human-Renin zwar ähnlich, aber nicht identisch ist.

Die bei den monoklonalen Antikörpern R 3—21, R 2—1 und R 3—27 beobachtete bloss partielle Hemmung von Human-Renin auch bei hohen Konzentrationen (Tabelle 1, letzte Spalte) wird gleichermassen auch für Marmoset-Renin gefunden.

## Beispiel 6

Charakterisierung der monoklonalen Antikörper

6.1 Bestimmung der Antikörper-Klasse

Die Klasse bzw. Unterklasse der von klonierten Hybridoma-Zellen produzierten monoklonalen Antikörper wird in einem ELISA bestimmt. Mikrotiterplatten werden mit je 1 µg einer Kaninchen-Immunglobulin-Präparation eines Klassen- bzw. Unterklassen-spezifischen Serums in 50 µl PBS beschichtet, freie Bindungsstellen der Platte mit RIA-Puffer (1% BSA in PBS, 0,2% NaN₃, pH 7,4) abgesättigt und die zu analysierenden Proben während 1 Std. bei 37°C in den Vertiefungen inkubiert. Nach Waschen der Platte werden die gebundenen monoklonalen Maus-Antikörper mit einer Präparation von Phosphatase-markiertem Kaninchen-Immunglobulin der für die Platten-Beschichtung verwendeten Serum-Präparation 1 Std. bei 37°C inkubiert und die aufgenommene Menge Enzym wie in Beispiel 4.1 bestimmt. Die Resultate sind in Tabelle 1 aufgeführt.

6.2 Analyse der Aminosäuresequenz

Die monoklonalen Antikörper werden mit 4-Dimethylamino-4'-iodacetamido-azobenzol derivatisiert und leichte und schwere Ketten auf einer Sepharose® G 100 Säule chromatographisch getrennt. Die Fraktionen werden durch Elektrodialyse vom Gel eluiert und im Beckman 8906 Sequenzer in bekannter Weise [9] einer Aminosäuresequenz-Analyse unterworfen.

Die N-terminale Aminosäuresequenz der leichten Kette des monoklonalen Antikörpers R 3—36—16 (umfassend die erste hypervariable Region in Position 28—38) lautet:

1 10 20
Asp-Asn-Val-Leu-Thr-Gln-Ser-Pro-Ser-Ser-Leu-Ala-Val-Ser-Leu-(Arg)-Gln-(Arg)-Ala-Thr-Ile-Ser-Cys-(Arg)-
30 40
Ala-Ser-Glu-Ser-Val-(Asp)-Ser-Tyr-Gly-Lys-X-Phe-Met-Y-Trp-Tyr

Eingeklammerte Aminosäuren sind nicht mit Sicherheit bekannt. X und Y stellen nicht-bestimmte Aminosäuren dar.

Beispiel 7
Bestimmung der Kreuzinhibition der monoklonalen Antikörper

Mikrotiterplatten werden analog Beispiel 4.1 mit monoklonalen Antikörpern der Gruppen 1 bis 3 (Tabelle 1) derart beschichtet, dass pro Vertiefung ca. 150 ng monoklonale Antikörper gebunden werden. 3 μg (20-facher Ueberschuss) des gleichen monoklonalen Antikörpers oder eines anderen monoklonalen Antikörpers der Gruppen 1 bis 3 werden mit 5 ng [125]I-markiertem Renin (Beispiel 8.1) in 10 μl Lösung 15 Std. bei 4°C oder 4 Std. bei 37°C vorinkubiert, dann in den Vertiefungen der beschichteten Mikrotiterplatte 2 Std. bei 37°C und 15 Std. bei 4°C inkubiert. Die Platten werden anschliessend gewaschen und das gebundene Renin durch Messung der Radioaktivität bestimmt.

Die Resultate dieses Kreuzinhibitions-Experiments sind in Tabelle 2 angegeben. Bindung von Renin an einen Oberflächen-gebundenen monoklonalen Antikörper wird durch Vorinkubation mit dem gleichen monoklonalen Antikörper in Lösung vollständig (>95%) inhibiert (Tabelle 2, Diagonale). Die monoklonalen Antikörper R 3—36—16 und R 3—47 inhibieren sich gegenseitig vollständig, erkennen also dasselbe Epitop auf dem Renin-Molekül. Der monoklonale Antikörper R 3—21, welche mit R 3—27 kreuzreagiert, wird ebenfalls vom monoklonalen Antikörper R 3—17—7 kreuzweise inhibiert, hingegen beeinflussen sich R 3—27 und R 3—17—7 gegenseitig nicht. Diese drei monoklonalen Antikörper erkennen somit teilweise überlappende, aufeinanderfolgende Epitope auf dem Renin-Molekül. Alle übrigen monoklonalen Antikörper-Paarungen zeigen keine gegenseitige Beeinflussung. Diese monoklonalen Antikörper binden somit räumlich getrennt angeordnete Epitope auf dem Renin-Molekül.

### Tabelle 2: Kreuzinhibition der monoklonalen Antikörper

| Auf Titerplatte gebundener monoklonaler Antikörper | Inhibition der Bindung an Renin durch überschüssigen monoklonalen Antikörper | | | | | | |
|---|---|---|---|---|---|---|---|
| | R 2-12 | R 3-17-7 | R 3-21 | R 3-27 | R 2-1 | R 3-36 -16 | R 3-47 |
| R 2-12 | +++ | – | – | – | – | – | – |
| R 3-17-7 | – | +++ | +++ | – | – | – | – |
| R 3-21 | – | ++ | +++ | + | – | – | – |
| R 3-27 | – | – | +++ | +++ | – | – | – |
| R 2-1 | – | – | – | – | +++ | – | – |
| R 3-36-16 | – | – | – | – | – | +++ | +++ |
| R 3-47 | – | – | – | – | – | +++ | +++ |

– 0-30% Inhibition
+ 30-60% Inhibition
++ 60-95% Inhibition
+++ 95-100% Inhibition

Beispiel 8
Radioimmunoassay (RIA) zur Bestimmung von Human-Renin in Plasma
8.1 Markierung von Human-Renin mit [125]I

10 μg Human-Renin aus Beispiel 1 werden nach dem Standardverfahren von Greenwood und Hunter [10] mit [125]I-Natriumiodid (0,3 mC) und Chloramin T iodiert. Das Reaktionsprodukt wird über eine Ionenaustauschersäule Bio Rad AG® 1×8 gereinigt und mit PBS auf eine Aktivität von 1,6×10⁶ cpm/ml eingestellt.

**8.2 Bestimmung von Human-Renin durch einfachen RIA**

Polyvinylchlorid-Mikrotiterplatten (Dynatech Labs. Inc.) werden durch Inkubation mit 100 µl einer Lösung in PBS, die je 10 µg/ml monoklonalen Antikörper R 2—1 und R 3—27 enthält, während 2 Std. bei 37°C und 15 Std. bei 4°C beschichtet. Die Platten werden mit PBS gewaschen und noch vorhandene Protein-reaktive Stellen durch Inkubation mit 225 µl RIA-Puffer (1% BSA in PBS, 0,2% NaN$_3$, pH 7,4) während 2 Std. bei 37°C abgesättigt.

Je 50 µl von Verdünnungsreihen einer Probelösung oder der Renin-Standardlösung in RIA-Puffer enthaltend 10% Renin-freies menschliches Plasma werden in die Vertiefungen der Mikrotiterplatten gegeben, mit je 50 µl (80'000 cpm entsprechend 2,5 ng) der Lösung von $^{125}$I-markiertem Renin aus Beispiel 8.1 versetzt und 2 Std. bei 37°C und 15 Std. bei 4°C inkubiert. Die Platten werden mit PBS gewaschen und die Radioaktivität gemessen. Die Konzentration von Human-Renin in der Probelösung wird durch eine mit Standardlösung erstellte Eichkurve bestimmt.

Auf gleiche Weise werden Mikrotiterplatten mit einem monoklonalen Antikörper oder mit zwei monoklonalen Antikörpern, die verschiedene Epitope erkennen (Tabelle 2), beschichtet und für einen einfachen RIA verwendet.

**8.3 Markierung des monoklonalen Antikörpers R 3—36—16 mit $^{125}$I**

30 µg monoklonaler Antikörper R 3—36—16 werden analog Beispiel 8.1 mit $^{125}$I (1 mC) iodiert und gereinigt.

Auf gleiche Weise wird der monoklonale Antikörper R 3—27 markiert.

**8.4 Bestimmung von Human-Renin durch Sandwich-RIA**

Analog Beispiel 8.2 werden Mikrotiterplatten mit 150 µl einer Lösung des monoklonalen Antikörpers R 3—27 (10 µg/ml) beschichtet und Protein-reaktive Stellen abgesättigt. Je 50 µl von Verdünnungsreihen einer Probelösung (z.B. Blutplasma eines Patienten) oder der Renin-Standardlösung in Renin-freiem menschlichem Plasma, 50 µl RIA-Puffer und 50 µl (120'000 cpm entsprechend 6 ng) der Lösung von $^{125}$I-markiertem Antikörper R 3—36—16 aus Beispiel 8.3 werden in den Vertiefungen der Mikrotiterplatten 2 Std. bei 37°C und 15 Min. bei 4°C inkubiert. Die Platten werden mit PBS gewaschen und die Radioaktivität gemessen. Die Konzentration von Human-Renin in der Probelösung wird durch eine mit Standardlösung erstellte Eichkurve (Figur 1) bestimmt.

Die Empfindlichkeit des Tests erlaubt die Bestimmung von nur 1 pg Human-Renin in 50 µl Probelösung (20 pg/ml). Eine lineare Dosisabhängigkeit ist im gesamten Bereich von 1 bis 100 pg Renin (50 µl Probe) gegeben.

Auf gleiche Weise kann Human-Renin ebenfalls bestimmt werden, wenn für die Beschichtung der Mikrotiterplatten z.B. der monoklonale Antikörper R 2—1 oder ein Gemisch von R 2—1 und R 3—27 verwendet werden, oder wenn die Antikörper vertauscht, d.h. der monoklonale Antikörper R 3—36—16 für die Beschichtung und z.B. der $^{125}$I-markierte monoklonale Antikörper R 3—27 für die Entwicklung verwendet werden.

**8.5 Test Kit für einfachen RIA**

Ein Test-Kit für in 8.2 beschribenen einfachen RIA enthält:
Mikrotiterplatten aus Polyvinylchlorid
    2 ml Lösung der monoklonalen anti-Human-Renin-Antikörper R 2—1 und R 3—27 (je 10 µg/ml)
    100 ml phosphatgepufferte physiologische Salzlösung (PBS)
    100 ml RIA-Puffer (1% BSA in PBS, 0,2% Natriumazid)
    2 ml Renin-freies menschliches Plasma
    2 ml Lösung von $^{125}$I-markiertem Human-Renin der Aktivität 1,6×10$^6$ cpm/ml
    2 ml Standardlösung enthaltend 20 ng/ml Human-Renin in Renin-freiem menschlichem Plasma
Eichkurve

**8.6 Test-Kit für Sandwich-RIA**

Ein Test-Kit für den in 8.4 beschriebenen Sandwich-RIA enthält:
Mikrotiterplatten aus Polyvinylchlorid
    6 ml Lösung der monoklonalen anti-Human-Renin-Antikörper R 3—27 (10 µg/ml)
    100 ml phosphatgepufferte physiologische Salzlösung (PBS)
    100 ml RIA-Puffer (1% BSA in PBS, 0,2% Natriumazid)
    2 ml Renin-freies menschliches Plasma
    2 ml Lösung von $^{125}$I-markiertem monoklonalem anti-Human-Renin Antikörper R 3—36—16 (Beispiel 8.3) der Aktivität 2,4×10$^6$ cpm/ml
    2 ml Standardlösung enthaltend 20 ng/ml Human-Renin in Renin-freiem menschlichem Plasma
Eichkurve (Figur 1)

Beispiel 9

Enzymimmuno-Assay (ELISA) zur Bestimmung von Human-Renin in Plasma

9.1 ELISA mit polyklonalem Anti-Human-Renin-Serum

Analog Beispiel 8.2 werden Mikrotiterplatten mit 100 µl einer Lösung enthaltend 10 µg/ml monoklonaler Antikörper R 3—36—16 beschichtet und Protein-reaktive Stellen abgesättigt. Die Platten werden mit je 50 µl von Verdünnungsreihen einer Probelösung oder einer Renin-Standardlösung in RIA-Puffer (1% BSA in PBS, 0,2% NaN₃, pH 7,4) enthaltend 10% Renin-freies menschliches Plasma 2 Std. bei 37°C inkubiert, gewaschen und dann mit 50 µl eines im Verhältnis 1:500 vorverdünnten polyklonalen Kaninchen-anti-Human-Renin-Serums [11] 1 Std. bei 37°C inkubiert. Nach Waschen der Platte werden die gebundenen Kaninchen-Antikörper durch Inkubation (1 Std. bei 37°C) mit einer im Verhältnis 1:1000 vorverdünnten Präparation von Phosphatase-markiertem Ziegenanti-Kaninchen-Immunglobulin entwickelt. Das gebundene Enzym setzt bei der weiteren Inkubation (30 Min., 37°C) mit 100 µl einer Lösung von p-Nitrophenylphosphat (1 mg/ml in 10% Diäthanolamin-Puffer enthaltend 0,5 mM MgCl₂, pH 9,8) p-Nitrophenol frei. Durch Messung der optischen Dichte bei 405 nm wird die Menge des freigesetzten p-Nitrophenol bestimmt, die proportional zur Menge des gebundenen Enzyms Phosphatase und damit proportional zur Menge Human-Renin in der Probelösung ist.

Auf gleiche Weise kann Human-Renin ebenfalls bestimmt werden, wenn für die Beschichtung der Mikrotiterplatten die monoklonalen Antikörper R 2—1, R 3—27 oder Mischungen zweier monoklonaler Antikörper verwendet werden.

9.2 Markierung des monoklonalen Antikörpers R 3—36—16 mit alkalischer Phosphatase

1,4 mg monoklonaler Antikörper R 3—36—16 in 1,4 ml PBS werden mit einer Lösung enthaltend 5 mg alkalische Phosphatase (SIGMA P6774, Typ VII—T) nach der Standard-Methode von Voller et al. [12] mit Glutaraldehyd (0,2% v/v) 2 Std. gekoppelt und in 5 ml Tris-Puffer 0.05 M, pH 8,0, enthaltend 1 mM MgCl₂, 1% BSA und 0,02% NaN₃ überführt. Die Lösung wird in der Dunkelheit bei 4°C aufbewahrt.

9.3 ELISA mit zwei verschiedenen monoklonalen Antikörpern

Polypropylen-Mikrotiterplatten (Dynatech Labs. Inc.) werden während 2 Stunden bei 37°C mit 150 µl einer Lösung des monoklonalen Antikörpers R 3—27 (10 µg/ml) in Puffer pH 8,6 (Carbonat-gepufferte 0,9% Kochsalzlösung enthaltend 0,02% Natriumazid) beschichtet. Die Platten werden fünfmal mit PBS gewaschen und noch vorhandene Proteinreaktive Stellen durch Inkubation mit 250 µl RIA-Puffer (1% BSA in PBS, 0,2% NaN₃, pH 7,4) während 1 Stunde bei 37°C abgesättigt. Derart beschichtete Platten können einige Tage in RIA-Puffer bei 4°C aufbewaht werden.

Je 50 µl von Verdünnungsreihen einer Probelösung (z.B. Blutplasma eines Patienten) oder dere Renin-Standardlösung in Renin-freiem menschlichem Plasma, 50 µl RIA-Puffer und 50 µl einer mit RIA-Puffer 1:100 verdünnten Lösung des Phosphatase-markierten Antikörpers R3—36—16 (Beispiel 9.2) werden gemischt und in den Vertiefungen der Mikrotiterplatten 2 Stunden bei 37°C und 30 Min. bei 4°C inkubiert. Die Platten werden fünfmal mit PBS gewaschen und die gebundene Menge Enzym nach Inkubation (30 Min., 37°C) mit 150 µl einer Lösung von p-Nitrophenylphosphat wie in Beispiel 9.1 bestimmt. Die Konzentration von Human-Renin in der Probelösung wird durch eine mit Standardlösung erstellte Eichkurve (Figur 2) errechnet.

Die Empfindlichkeit des ELISA erlaubt die Bestimmung von weniger als 1 pg Human-Renin in 50 µl Probelösung (20 pg/ml). Eine lineare Dosisabhängigkeit ist im Bereich von 1 bis 100 pg Renin (50 µl Probe) gegeben.

9.4 Test-Kit für ELISA

Ein Test-Kit für den in Beispiel 9.3 beschriebenen ELISA enthält:

Mikrotiterplatten aus Polypropylen,

6 ml Lösung des monoklonalen anti-Human-Renin-Antikörpers R 3—27 (10 µg/ml) in Carbonat-gepufferter (0,072 g/l Na₂CO₃ und 4,2 g/l NaHCO₃) 0,9% Kochsalzlösung enthaltend 0,02% NaN₃,

100 ml phosphatgepufferte physioligische Salzlösung (PBS),

100 ml RIA-Puffer (1% BSA in PBS, 0,2% NaN₃),

2 ml Renin-freies menschliches Plasma,

0,2 ml Lösung von mit alkalischer Phosphatase markiertem monoklonalem anti-Human-Renin-Antikörper R 3—36—16 (Beispiel 9.2) in Tris-Puffer (0,05 M, pH 8,0, 1 mM MgCl₂, 1% BSA, 0,02% NaN₃) der Konzentration 0,3 mg Antikörper pro ml,

10 ml Lösung von p-Nitrophenylphosphat (1 mg/ml) in Diäthanolaminpuffer (10% Diäthanolamin, 0,5 mM MgCl₂, 0,02% NaN₃, mit HCl auf pH 8,9 eingestellt),

2 ml Standardlösung enthaltend 20 ng/ml Human-Renin in Renin-freiem menschlichem Plasma,

Eichkurve (Figur 2),

Farbstärkenskala zur Bestimmung der Menge freigesetzten p-Nitrophenols mit blossem Auge.

EP 0 158 599 B1

Beispiel 10

Immuno-Affinitätschromatographie zur Reinigung von Renin

10.1 Herstellung von Immuno-Affinitäts-Gel

Affi-Gel® 10 (Bio-Rad) wird nach den Angaben des Herstellers mit kaltem destilliertem Wasser und Kupplungspuffer pH 8,0 (0,1 M NaHCO$_3$-Lösung) gewaschen. Eine 50 % ige Suspension des Gels in Kupplungspuffer (1 ml) wird in ein Plastikrohr übertragen, mit dem gleichen Volumen einer gereinigten monoklonalen Antikörper-Lösung, welche 10 mg Protein enthält, vermischt und 4 Std. bei Zimmertemperatur rotiert. Danach wird das Gel mit Kupplungspuffer gewaschen. Zur Blockierung der noch freien aktiven Stellen wird das Gel mit 0,1 ml 1 M Aethanolamin-HCl (pH 8,0) pro ml Gel während 2 Std. bei Raumtemperatur behandelt, dann mit PBS (enthaltend 10 mM Natriumazid) gewaschen und darin bei 4°C gehalten. Der Kupplungsgrad wird durch Messung der optischen Dichte bei 280 nm bestimmt und beträgt ca. 8 mg monoklonaler Antikörper pro ml Gel.

10.2 Reinigung von Marmoset- oder Human-Renin

100 µl eines mit dem monoklonalen Antikörper R 2—1 nach Beispiel 10.1 hergestellten Immuno-Affinitäts-Gels werden mit 1 ml Nierenextrakt aus Marmosets (Callitrix jacchus) in einem 5 ml-Falcon-Röhrchen während 30 Min. bei Raumtemperatur unter periodischen Schüttelbewegungen inkubiert. Der Ueberstand wird entfernt, das Gel mit PBS gewaschen und mit 1 ml Glycinhydrochlorid-Puffer pH 3,0 extrahiert. Die Extrakte werden mit Tris-Puffer pH 8,5 neutralisiert. Auf diese Weise werden 70% (5—6 ng) des im Rohextrakt vorhandenen Renins rein erhalten.

Auf gleiche Weise kann Human-Renin gereinigt werden.

Analog können Immuno-Affinitäts-Gele enthaltend die monoklonalen Antikörper R 3—21, R 1—19 oder R 1—20 zur Reinigung von Human- oder Marmoset-Renin eingesetzt werden.

Beispiel 11

Pharmazeutisches Präparat für parenterale Verabreichung

7,0 mg monoklonaler Antikörper R 3—36—16 hergestellt nach Beispiel 3 werden in 20 ml physioligischer Kochsalz-Lösung gelöst. Die Lösung wird durch ein bakteriologisches Filter geleitet, das Filtrat aufgeteilt und unter aseptischen Bedingungen in 10 Ampullen abgefüllt. Die Ampullen enthalten je 0,7 mg monoklonalen Antikörper geeignet für parenterale Verabreichung und werden bevorzugt in der Kälte, beispielsweise bei −20°C, gelagert.

Literaturzitate

[1] G. Köhler und C. Milstein, Nature 256, 495 (1975).

[2] D. Simon, F. X. Galen, C. Devaux, F. Soubrier, B. Pau, J. Menard und P. Corvol, J. Clin. Endocr. Met. 53 453 (1981).

[3] V. J. Dzau, D. Devine, M. Mudgett-Hunter, R. I. Kopelman, A. C. Barger und E. Haber, Clinical and Experimental Hypertension A5, 1207 (1983).

[4] F. Soubrier, T. T. Guyenne, M. F. Gonzales, P. Corvol und J. Ménard, in "Heterogeneity of Renin and Renin-Substrate" (Herausg. M. P. Sambhi), Elsevier North Holland Inc. 1981, S. 237.

[5] D. R. Bangkan, I. Robertson, J. I. S. Robertson, C. J. Robinson und M. Iree, Clin. Sci. Mol. Med. 48, 135 (1975).

[6] B. Pau, D. Simon, F. X. Galen, C. Devaux, F. Soubrier, J. Ménard und P. Corvol, Clin. Sci. 61 239 (1981).

[7] M. Shulman, C. D. Wilde und G. Köhler, Nature 276, 269 (1978).

[8] E. Engvall und P. Perlmann, J. Immunol. 109 129 (1972).

[9] J. Y. Chang. H. Herbst, R. Aebersold und D. G. Braun, Biochem. J. 211, 173 (1983).

[10] F. C. Greenwood, W. M. Hunter und J. S. Glover, Biochem. J. 89 114 (1963).

[11] F. X. Galen, T. T. Guyenne, C. Devaux, C. Auzan, P. Corvol und J. Ménard, J. Clin. Endocr. Met. 48, 1041 (1979).

[12] A. Voller, D. E. Bidwell und A. Barlett, Bull. World Health Organ. 53, 55 (1976).

**Patentansprüche für die Vertragstaaten: DE CH LI GB FR IT NL BE SE LU**

1. Monoklonale Antikörper gegen Human-Renin und strukturell ähnlichem Renin, sowie Derivate davon, dadurch gekennzeichnet, dass sie bei einer Konzentration von $2\times10^{-8}$ M (Mol/Liter) die Enzym-Aktivität von Human-Renin zu mindestens 50% hemmen.

2. Monoklonale Antikörper und Derivate davon gemäss Anspruch 1, dadurch gekennzeichnet, dass sie zusätzlich bei einer Konzentration von $3\times10^{-10}$M mindestens 50% Human-Renin, das in limitierender Menge zugesetzt ist, binden.

3. Monoklonale Antikörper und Derivate davon gemäss Anspruch 1, dadurch gekennzeichnet, dass sie bei einer Konzentration von $2\times10^{-9}$M die Enzym-Aktivität von Human-Renin zu mindestens 50% hemmen.

4. Monoklonale Antikörper und Derivate davon gemäss Anspruch 1, dadurch gekennzeichnet, dass sie bei einer Konzentration von $5\times10^{-11}$M die Enzym-Aktivität von Human-Renin zu mindestens 50% hemmen.

5. Der monoklonale Antikörper R 3—36—16, dadurch gekennzeichnet, dass der monoklonale Antikörper von der Hybridoma-Zellinie mit der Bezeichnung R 3—36—16 produziert wird, die in der "Collection Nationale de Cultures de Microorganismes" des Institut Pasteur in Paris unter der Nummer I—253 hinterlegt ist.

6. Mit $^{125}$I markierte monoklonale Antikörper-Derivate gemäss einem der Ansprüche 1 bis 4.

7. Das mit $^{125}$I markierte Derivat des monoklonalen Antikörpers R 3—36—16 gemäss Anspruch 5.

8. Mit alkalischer Phosphatase konjugierte monoklonale Antikörper-Derivate gemäss einem der Ansprüch 1 bis 4.

9. Das mit alkalischer Phosphatase konjugierte Derivat des monoklonalen Antikörpers R 3—36—16 gemäss Anspruch 5.

10. Verfahren zur Herstellung von monoklonalen Antikörpern und Derivaten davon gemäss Anspruch 1, dadurch gekennzeichnet, dass man solche monoklonale Antikörper produzierende Hybridoma-Zellen

a) in vitro kultiviert und aus den Kulturüberständen die monoklonalen Antikörper isoliert, oder

b) in vivo in einem geeigneten Säugetier vermehrt und aus den Körperflüssigkeiten dieses Säugetiers die monoklonalen Antikörper isoliert, und, wenn erwünscht,

c) einen erhaltenen monoklonalen Antikörper in ein Derivat davon überführt.

11. Verfahren gemäss Anspruch 10, dadurch gekennzeichnet, dass man gewünschte monoklonale Antikörper produzierende Hybridoma-Zellen abstammend von Balb/c-Mäusen intraperitoneal in gegebenenfalls mit einem Kohlenwasserstoff vorbehandelte Balb/c-Mäuse injiziert, nach 8—10 Tagen diesen Tieren Aszitesflüssigkeit entnimmt und daraus die monoklonalen Antikörper durch Fällung mit Ammoniumsulfat und chromatographische Reinigung isoliert.

12. Hybridoma-Zellinien, dadurch gekennzeichnet, dass sie monoklonale Antikörper gemass einem der Ansprüche 1 bis 5 produzieren.

13. Die Hybridoma-Zellinie gemäss Anspruch 12 mit der Bezeichnung R 3—36—16, die in der "Collection Nationale der Cultures de Microorganismes" des Institut Pasteur in Paris unter der Nummer I—253 hinterlegt ist.

14. Verfahren zur Herstellung von Hybridoma-Zellinien gemäss Anspruch 12, dadurch gekennzeichnet, dass man geeignete Säugetiere mit gereinigtem Human-Renin immunisiert, dem Säugetier entnommene Antikörper-produzierende Zellen mit Myeloma-Zellen fusioniert, die erhaltenen Hybridoma-Zellen kloniert und diegenigen Zellklone, die die gewünschten monoklonalen Antikörper mit hoher Affinität zu Human-Renin produzieren, selektioniert, indem man die Zellüberstände auf Bindung von monoklonalen Antikörpern an Oberflächen-fixiertes Human-Renin, Bindung an gelöstes Human-Renin und auf Hemmung der Enzym-Aktivität von Human-Renin prüft.

15. Verfahren gemäss Anspruch 14, dadurch gekennzeichnet, dass man Balb/c-Mäuse durch drei bis sechs Injektionen parenteral mit je zwischen 1 μg bis 20 μg gereinigtem Human-Renin in Abstanden von ein bis sechs Wochen immunisiert, zwei bis sechs Tage nach der letzten ("booster") Immunisierung den Tieren Milzzellen entnimmt und diese mit Myeloma-Zellen einer geeigneten Zellinie in Gegenwart eines Fusions-Promotors fusioniert.

16. Verfahren gemäss Anspruch 14, dadurch gekennzeichnet, dass man monoklonale Antikörper-produzierende Zellen von Balb/c-Mäusen mit Myeloma-Zellen der Zellinien X63-Ag8.653 oder Sp2/O-Ag14 fusioniert.

17. Verfahren gemäss Anspruch 14, dadurch gekennzeichnet, dass man Myeloma-Zellen mit einem drei- bis zwanzigfachen Ueberschuss an Milzzellen aus immunisierten Tieren in einer Lösung enthaltend 30 bis 50% Polyäthylenglykol eines Molekulargewichts zwischen 1000 und 4000 fusioniert.

18. Verwendung der monoklonalen Antikörper und/oder ihrer Derivate gemäss einem der Ansprüch 1 bis 9 zur qualitativen und quantitativen Bestimmung von Human-Renin und strukturell ähnlichem Renin *in vitro*.

19. Verwendung der monoklonalen Antikörper und Derivate in einem Radioimmunoassay gemäss Anspruch 18.

20. Verwendung der monoklonalen Antikörper und Derivate in einem Enzym-Immunoassay gemäss Anspruch 18.

21. Test-Kits zur Bestimmung von Human-Renin und strukturell ähnlichem Renin enthaltend monoklonale Antikörper und/oder Derivate davon gemäss einem der Ansprüche 1 bis 9 und gegebenenfalls Hilfsmittel.

22. Test-Kits gemäss Anspruch 21 für einen Radioimmunoassay.

23. Test-Kits gemäss Anspruch 21 für einen Enzym-Immunoassay.

24. Verwendung der monoklonalen Antikörper oder ihrer Derivate gemäss einem der Ansprüche 1 bis 5 zur Reinigung von Human-Renin und strukturell ähnlichem Renin.

25. Pharmazeutische Präparate enthaltend einen monoklonalen Antikörper oder ein Derivat davon gemäss einem der Ansprüche 1 bis 5 und gegebenenfalls pharmazeutische Hilfsmittel.

26. Verwendung der monoklonalen Antikörper oder Derivate davon gemäss einem der Ansprüche 1 bis 5 zur Herstellung eines pharmazeutischen Präparats zur Behandlung von hohem Blutdruck und Herzinsuffizienz.

# EP 0 158 599 B1

**Patentansprüche für den Vertragsstaat: AT**

1. Verfahren zur Herstellung von monoklonalen Antikörpern gegen Human-Renin und strukturell ähnliches Renin, die bei einer Konzentration von $2\times10^{-8}$m (Mol/Liter) die Enzym-Aktivität von Human-Renin zu mindestens 50% hemmen, sowie von Derivaten davon, dadurch gekennzeichnet, dass man solche monoklonale Antikörper produzierende Hybridoma-Zellen

a) in vitro kultiviert und aus den Kulturüberständen die monoklonalen Antikörper isoliert, oder

b) in vivo in einem geeigneten Säugetier vermehrt und aus den Körperflüssigkeiten dieses Säugetiers die monoklonalen Antikörper isoliert, und, wenn erwünscht,

c) einen erhaltenen monoklonalen Antikörper in ein Derivat davon überführt.

2. Verfahren gemäss Anspruch 1, dadurch gekennzeichnet, dass man gewünschte monoklonale Antikörper produzierende Hybridoma-Zellen abstammend von Balb/c-Mäusen intraperitoneal in gegebenenfalls mit einem Kohlenwasserstoff vorbehandelte Balb/c-Mäuse injiziert, nach 8—10 Tagen diesen Tieren Aszitesflüssigkeit entnimmt und daraus die monoklonalen Antikörper durch Fällung mit Ammoniumsulfat und chromatographische Reinigung isoliert.

3. Verfahren gemäss Anspruch 1, zur Herstellung von monoklonalen Antikörpern, die zusätzlich bei einer Konzentration von $3\times10^{-10}$M mindestens 50% Human-Renin, das in limitierender Menge zugesetzt ist, binden.

4. Verfahren gemäss Anspruch 1 zur Herstellung von monoklonalen Antikörpern, die bei einer Konzentration von $2\times10^{-9}$M die Enzym-Aktivität von Human-Renin zu mindestens 50% hemmen, sowie von Derivaten davon.

5. Verfahren gemäss Anspruch 1 zur Herstellung von monoklonalen Antikörpern, die bei einer Konzentration von $5\times10^{-11}$M die Enzym-Aktivität von Human-Renin zu mindestens 50% hemmen, sowie von Derivaten davon.

6. Verfahren zur Herstellung von monoklonalen Antikörpers R 3—36—16, der von der Hybridoma-Zellinie mit der Bezeichnung R 3—36—16 produziert wird, die in der "Collection Nationale de Cultures de Microorganismes" des Institut Pasteur in Paris unter der Nummer I-253 hinterlegt ist, dadurch gekennzeichnet, dass man die den Antikörper produzierenden Hybridoma-Zellen

a) in vitro kultiviert und aus den Kulturüberständen die monoklonalen Antikörper isoliert, oder

b) in vivo in einen geeigneten Säugetier vermehrt und aus den Körperflüssigkeiten dieses Säugetiers den monoklonalen Antikörper isoliert, und, wenn erwünscht,

c) den erhaltenen monoklonalen Antikörper in ein Derivat davon überführt.

7. Verfahren gemäss Anspruch 1 zur Herstellung von $^{125}$I-markierten monoklonalen Antikörper-Derivaten, dadurch gekennzeichnet, dass der monoklonale Antikörper in Schritt c) mit $^{125}$I-Natrium- oder Kaliumiodid und einem chemischen oder enzymatischen Oxidationsmittel umgesetzt wird.

8. Verfahren gemäss Anspruch 6 sur Herstellung eines $^{125}$I-markierten Derivats des monoklonalen Antikörpers R 3—36—16.

9. Verfahren gemäss Anspruch 1 zur Herstellung von mit alkalischer Phosphatase konjugierten monoklonalen Antikörper-Derivaten, dadurch gekennzeichnet, dass der monoklonale Antikörper in Schritt c) mit alkalischer Phosphatase und einem Kupplungsreagens umgesetzt wird.

10. Verfahren gemäss Anspruch 6 zur Herstellung eines mit alkalischer Phosphatase konjugierten Derivats des monoklonalen Antikörpers R 3—36—16.

11. Hybridoma-Zellinien, dadurch gekennzeichnet, dass sie monoklonale Antikörper gegen Human-Renin und Strukturell ähnliches Renin produzieren, die bei einer Konzentration von $2\times10^{-8}$ M die Enzym-Aktivität von Human-Renin zu mindestens 50% hemmen.

12. Hybridoma-Zellinien gemäss Anspruch 11, dadurch gekennzeichnet, dass sie monoklonale Antikörper produzieren, die zusätzlich bei einer Konzentration von $3\times10^{-10}$M mindestens 50% Human-Renin, das in limitierender Menge zugesetzt ist, binden.

13. Hybridoma-Zellinien gemäss Anspruch 11, dadurch gekennzeichnet, dass sie monoklonale Antikörper produzieren, die bei einer Konzentration von $2\times10^{-9}$M die Enzym-Aktivität von Human-Renin zu mindestens 50% hemmen.

14. Hybridoma-Zellinien gemäss Anspruch 11, dadurch gekennzeichnet, dass sie monoklonale Antikörper produzieren, die bei einer Konzentration von $5\times10^{-11}$M die Enzym-Aktivität von Human-Renin zu mindestens 50% hemmen.

15. Die Hybridoma-Zellinie mit der Benzeichnung R 3—36—16, die in der "Collection Nationale de Cultures de Microorganismes" des Institut Pasteur in Paris unter der Nummer I—253 hinterlegt ist.

16. Verfahren zur Herstellung von Hybridoma-Zellinien gemäss Anspruch 11 oder 15, dadurch gekennzeichnet, dass man geeignete Säugetiere mit gereinigtem Human-Renin immunisiert, dem Säugetier entnommene Antikörper-produzierende Zellen mit Myeloma-Zellen fusioniert, die erhaltenen Hybridoma-Zellen kloniert und diegenigen Zellklone, die die gewünschten monoklonalen Antikörper mit hoher Affinität zu Human-Renin produzieren, selektioniert, indem man die Zellüberstände auf Bindung von monoklonalen Antikörpern an Oberflächen-fixiertes Human-Renin, Bindung an gelöstes Human-Renin und auf Hemmung der Enzym-Aktivität von Human-Renin prüft.

17. Verfahren gemäss Anspruch 16, dadurch gekennzeichnet, dass man Balb/c-Mäuse durch drei bis sechs Injektionen parenteral mit je zwischen 1 μg bis 20 μg gereinigtem Human-Renin in Abständen von ein bis sechs Wochen immunisiert, zwei bis sechs Tage nach der letzten ("booster") Immunisierung den Tieren Milzzellen entnimmt und diese mit Myeloma-Zellen einer geeigneten Zellinie in Gegenwart eines Fusions-Promotors fusioniert.

18. Verfahren gemäss Anspruch 16, dadurch gekennzeichnet, dass man monoklonale Antikörper-produzierende Zellen von Balb/c-Mäusen mit Myeloma-Zellen der Zellinien X63-Ag8.653 oder Sp2/O-Ag14 fusioniert.

19. Verfahren gemäss Anspruch 16, dadurch gekennzeichnet, dass man Myeloma-Zellen mit einem drei- bis zwanzigfachen Ueberschuss an Milzzellen aus immunisierten Tieren in einer Lösung enthaltend 30 bis 50% Polyäthylenglykol eines Molekulargewichts zwischen 1000 und 4000 fusioniert.

## Revendications pour les Etats contractants: BE CH DE FR GB IT LI LU NL SE

1. Anticorps monoclonaux contre la rénine humaine et la rénine structurellement apparentée, ainsi que leurs dérivés, caractérisés en ce qu'ils inhibent à une concentration de $2 \times 10^{-8}$M (moles/litre) l'activité enzymatique de la rénine humaine à au moins 50%.

2. Anticorps monoclonaux et leurs dérivés selon la revendication 1, caractérisés en ce qu'en outre ils lient à une concentration de $3 \times 10^{-10}$M au moins 50% de rénine humaine qui est ajoutée en quantité limitante.

3. Anticorps monoclonaux et leurs dérivés selon la revendication 1, caractérisés en ce qu'ils inhibent à une concentration de $2 \times 10^{-9}$M l'activité enzymatique de la rénine humaine à au moins 50%.

4. Anticorps monoclonaux et leurs dérivés selon la revendication 1, caractérisés en ce qu'ils inhibent à une concentration de $5 \times 10^{-11}$M l'activité enzymatique de la rénine humaine à au moins 50%.

5. Anticorps monoclonal R 3—36—16, caractérisé en ce que l'anticorps monoclonal est produit à partir de la lignée cellulaire d'hybridome désignée par R 3—36—16 qui est déposée dans la "Collection Nationale de Cultures de Microorganismes" de l'Institut Pasteur à Paris sous le n° I—253.

6. Dérivés d'anticorps monoclonaux marqués avec$^{125}$I selon l'une des revendications 1 à 4.

7. Dérivé marqué avec$^{125}$I de l'anticorps monoclonal R 3—36—16 selon la revendication 5.

8. Dérivés d'anticorps monoclonaux conjugués avec la phosphatase alcaline selon l'une des revendications 1—4.

9. Dérivé de l'anticorps monoclonal R 3—36—16 selon la revendication 5, conjugué avec la phosphatase alcaline.

10. Procédé de préparation d'anticorps monoclonaux et de leurs dérivés selon la revendiction 1, caractérisé en ce que ces cellules hybridomes productrices d'anticorps monoclonaux sont

a) cultivées *in vitro* avec isolement des anticorps monoclonaux à partir des surnageants de culture, ou

b) multipliées *in vivo* chez un mammifère approprié avec isolement des anticorps monoclonaux à partir des liquides corporels de ces mammifères et, si on le désire,

c) en ce qu'un anticorps monoclonal obtenu est transformé en un de ses dérivés.

11. Procédé selon la revendication 10, caractérisé en ce qu'on injecte des cellules hybridomes productrices d'anticorps monoclonaux désirés et provenant de souris Balb/c par voie intrapéritonéale à des souris Balb/c éventuellement prétraitées avec un hydrocarbure, en ce qu'au bout de 8 à 10 jours, on prélève le liquide d'ascite de ces animaux et en ce qu'on en isole les anticorps monoclonaux par précipitation avec du sulfate d'ammonium et purification chromatographique.

12. Lignées cellulaires d'hybridomes caractérisées en ce qu'elles produisent des anticorps monoclonaux selon l'une des revendications 1—5.

13. La lignée cellulaire d'hybridome selon la revendication 12 ayant la désignation R 3—36—16 déposée dans la "Collection Nationale de Cultures de Microorganismes" de l'Institut Pasteur à Paris sous le n° I—253.

14. Procédé de préparation de lignées cellulaires d'hybridomes selon la revendication 12, caractérisé en ce qu'on immunise des mammifères appropriés avec de la rénine humaine purifiée, en ce qu'on fusionne les cellules productrices d'anticorps prélevées chez le mammifère avec des cellules de myélome, en ce qu'on clone les cellules hybridomes obtenues et en ce qu'on sélectionne les clones cellulaires qui produisent les anticorps monoclonaux désirés ayant une affinité élevée à la rénine humaine, en testant les surnageants cellulaires quant à la liaison des anticorps monoclonaux à la rénine humaine fixée aux surfaces, à la liaison à la rénine humaine dissoute et à l'inhibition de l'activité enzymatique de la rénine humaine.

15. Procédé selon la revendication 14, caractérisé en ce qu'on immunise des souris Balb/c par 3 à 6 injections parentérales avec à chaque fois entre 1 µg et 20 µg de rénine humaine purifiée à des intervalles de 1 à 6 semaines, en ce que deux à six jours après la dernière immunisation ("rappel") on prélève des cellules de rate des animaux et en ce qu'on les fusionne avec des cellules de myélome d'une lignée cellulaire appropriée en présence d'un promoteur de fusion.

16. Procédé selon la revendication 14, caractérisé en ce qu'on fusionne des cellules productrices d'anticorps monoclonaux de souris Balb/c avec des cellules de myélome des lignées cellulaires X63—Ag8.653 ou Sp2/O—Ag14.

17. Procédé selon la revendication 14, caractérisé en ce qu'on fusionne des cellules de myélome avec un excès de trois à vingt fois de cellules de rate provenant d'animaux immunisés dans une solution contenant de 30 à 50% de polyéthylène glycol d'un poids moléculaire compris entre 1000 et 4000.

18. Application des anticorps monoclonaux et/ou de leurs dérivés selon l'une des revendications 1—6 à la détermination qualitative et quantitative de la rénine humaine et de la rénine structurellement apparentée *in vitro*.

19. Application des anticorps monoclonaux et de leurs dérivés dans un dosage radioimmunologique selon la revendication 18.

20. Application des anticorps monoclonaux et de leurs dérivés dans un dosage enzymoimmunologique selon la revendication 18.

21. Nécessaire expérimental pour la détermination de la rénine humaine et de la rénine structurellement apparentée contenant des anticorps monoclonaux et/ou leurs dérivés selon l'une des revendications 1—9 et éventuellement des adjuvants.

22. Nécessaires expérimentaux selon la revendication 21 pour un dosage radioimmunoligique.

23. Nécessaire expérimental selon la revendication 21 pour un dosage immunoenzymatique.

24. Application des anticorps monoclonaux ou de leurs dérivés selon l'une des revendications 1—5 à la purification de la rénine humaine et de la rénine structurellement apparentée.

25. Préparation pharmaceutique contenant un anticorps monoclonal ou l'un de ses dérivés selon l'une des revendications 1 à 5 et éventuellement des adjuvants pharmaceutiques.

26. Application des anticorps monoclonaux ou de leurs dérivés selon l'une des revendications 1—5 à la préparation d'une préparation pharmaceutique pour le traitement de l'hypertension artérielle et de l'insuffisance cardiaque.

**Revendications pour l'Etat contractant: AT**

1. Procédé de préparation d'anticorps monoclonaux contre la rénine humaine et la rénine structurellement apparentée, qui inhibent à une concentration de $2 \times 10^{-8}$M (mole/litre) l'activité enzymatique de la rénine humaine à au moins 50%, ainsi que de leurs dérivés, caractérisé en ce que ces cellules hybridomes productrices d'anticorps monoclonaux sont

a) cultivées *in vitro* avec isolement des anticorps monoclonaux à partir des surnagenants de culture, ou

b) multipliées *in vitro* chez un mammifère approprié avec isolement des anticorps monoclonaux à partir des liquides corporels de ces mammifères et, si on le désire,

c) en ce qu'un anticorps monoclonal obtenu est transformé en un de ses dérivés.

2. Procédé selon la revendication 1, caractérisés en ce qu'on injecte des cellules d'hybridomes productrices d'anticorps monoclonaux désirées dérivant de souris Balb/c par voie intrapéritonéale à des souris Balb/c éventuellement prétraitées avec un hydrocarbure, en ce qu'au bout de 8 à 10 jours, on prélève le liquide d'ascite de ces animaux et en ce qu'on en isole les anticorps monoclonaux par précipitation avec du sulfate d'ammonium et purification chromatographique.

3. Procédé selon la revendication 1, de préparation d'anticorps monoclonaux qui lient en outre à une concentration de $3 \times 10^{-10}$M au moins 50% de rénine humaine qui est ajoutée en quantité limitante.

4. Procédé selon la revendication 1, de préparation d'anticorps monoclonaux qui inhibent à une concentration de $2 \times 10^{-9}$M l'activité enzymatique de la rénine humaine à au moins 50%, ainsi que de leurs dérivés.

5. Procédé selon la revendication 1, de préparation d'anticorps monoclonaux qui inhibent à une concentration de $5 \times 10^{-11}$M l'activité enzymatique de la rénine humaine à au moins 50%, ainsi que de leurs dérivés.

6. Procédé de préparation de l'anticorps monoclonal R 3—36—16 qui est produit à partir de la lignée cellulaire d'hybridome désignée par R 3—36—16 déposée dans la "Collection Nationale de Cultures de Microorganismes" de l'Institut Pasteur à Paris sous le n° I—253, caractérisé en ce que les cellules d'hybridomes productrices d'anticorps sont

a) cultivées *in vitro* avec isolement des anticorps monoclonaux à partir des surnageants de culture, ou

b) multipliées de manière chez un mammifère approprié avec isolement des anticorps monoclonaux à partir des liquides corporels de ces mammifère et, si on le désire,

c) en ce que les anticorps monoclonaux obtenus transformés en un de leurs dérivés.

7. Procédé selon la revenication 1, de préparation de dérivés d'anticorps monoclonaux marqués avec $^{125}$I, caractérisé en ce que l'anticorps monoclonal dans l'étape c) est mis à réagir avec de l'iodure de $^{125}$I-sodium ou potassium et un oxydant chimique ou enzymatique.

8. Procédé selon la revendication 6, de préparation d'un dérivé marqué avec $^{125}$I de l'anticorps monoclonal R 3—36—16.

9. Procédé selon la revendication 1 de préparation de dérivés d'anticorps monoclonaux conjugués avec la phosphatase alcaline, caractérisé en ce qu'on fait réagir l'anticorps monoclonal dans l'étape c) avec la phosphatase alcaline et un réactif de couplage.

10. Procédé selon la revendication 6 de préparation d'un dérivé de l'anticorps monoclonal R 3—36—16 conjugué avec la phosphatase alcaline.

11. Lignées cellulaires d'hybridomes, caractérisées en ce qu'elles produisent des anticorps monoclonaux contre la rénine humaine et la rénine structurellement apparentée, qui inhibent à une concentration de $2 \times 10^{-8}$M l'activité enzymatique de la rénine humaine à au moins 50%.

12. Lignées cellulaires d'hybridomes selon la revendication 11, caractérisées en ce qu'elles produisent des anticorps monoclonaux qui lient en outre à une concentration de $3 \times 10^{10}$M au moins 50% de rénine humaine qui est ajoutée en quantité limitante.

13. Lignées cellulaires d'hybridomes selon la revendication 11, caractérisées en ce qu'elles produisent des anticorps monoclonaux qui inhibent à une concentration de $2 \times 10^{-9}$M l'activité enzymatique de la rénine humaine à au moins 50%.

14. Lignées cellulaires d'hybridomes selon la revendication 11, caractérisées en ce qu'elles produisent des anticorps monoclonaux qui inhibent à une concentration de $5 \times 10^{-11}$M l'activité enzymatique de la rénine humaine à au moins 50%.

15. Lignée cellulaire d'hybridomes désignée par R 3—36—16, qui est déposée dans la "Collection Nationale de Cultures de Microorganismes" de l'Institut Pasteur à Paris sous le n° I—253.

16. Procédé de préparation de lignées cellulaires d'hybridomes selon la revendication 11 ou 15, caractérisé en ce qu'on immunise des mammifères approppriés avec de la rénine humaine purifiée, en ce qu'on fusionne des cellules productrices d'anticorps prélevées chez le mammifère avec des cellules de myélome, en ce qu'on clone des cellules hybridomes obtenues et en ce qu'on sélectionne les clones cellulaires qui produisent l'anticorps monoclonal désiré ayant une affinité élevée pour la rénine humaine, en testant dans les surnageants cellulaires la liaison des anticorps monoclonaux à la rénine humaine fixée aux surfaces, à la liaison à la rénine humaine et l'inhibition de l'activité enzymatique de la rénine humaine.

17. Procédé selon la revendication 16, caractérisé en ce qu'on immunise des souris Balb/c par 3 à 6 injections par voie parentérale avec à chaque fois entre 1 µg et 20 µg de rénine humaine purifiée à des intervalles de 1 à 6 semaines, en ce qu'on prélève deux à six jours après la dernière immunisation ("rappel") chez les animaux des cellules de rate et en ce qu'on fusionne celles-ci avec des cellules de myélome d'une lignée cellulaire appropriée en présence d'un promoteur de fusion.

18. Procédé selon la revendication 16, caractérisé en ce qu'on fusionne des cellules productrices d'anticorps monoclonaux de souris Balb/c avec des cellules de myélome des lignées cellulaires X63—Ag8.653 ou Sp2/O—Ag14.

19. Procédé selon la revendication 16, caractérisé en ce qu'on fusionne des cellules de myélome avec un excès de 3 à 20 fois de cellules de rate provenant d'animaux immunisés dans une solution contenant de 30 à 50% de polyéthylène glycol d'un poids moléculaire compris entre 1000 et 4000.

## Claims for the Contracting States: BE CH DE FR GB IT LI LU NL SE

1. Monoclonal antibodies to human renin and structurally similar renin, and derivatives thereof, characterised in that, at a concentration of $2 \times 10^{-8}$ M (mol/litre), they inhibit the enzyme activity of human renin by at least 50%.

2. Monoclonal antibodies and derivatives thereof according to claim 1, characterised in that, at a concentration of $3 \times 10^{-10}$ M, they additionally bind at least 50% of human renin that has been added in a limiting amount.

3. Monoclonal antibodies and derivatives thereof according to claim 1, characterised in that, at a concentration of $2 \times 10^{-9}$ M, they inhibit the enzyme activity of human renin by at least 50%.

4. Monoclonal antibodies and derivatives thereof according to claim 1, characterised in that, at a concentration of $5 \times 10^{-11}$ M, they inhibit the enzyme activity of human renin by at least 50%.

5. Monoclonal antibody R 3—36—16, characterised in that the monoclonal antibody is produced by the hybridoma cell line designated R 3—36—16 which is deposited at the "Collection Nationale de Cultures de Microorganismes" of the Institut Pasteur in Paris under number I—253.

6. $^{125}$I-labelled monoclonal antibody derivatives according to any one of claims 1 to 4.

7. $^{125}$I-labelled derivative of the monoclonal antibody R 3—36—16 according to claim 5.

8. Monoclonal antibody derivatives according to any one of claims 1 to 4 conjugated with alkaline phosphatase.

9. Derivative of the monoclonal antibody R 3—36—16 according to claim 5 conjugated with alkaline phosphatase.

10. A process for the preparation of monoclonal antibodies and derivatives thereof according to claim 1, characterised in that hybridoma cells producing such monoclonal antibodies

a) are cultured in vitro and the monoclonal antibodies are isolated from the culture supernatants, or

b) are multiplied in vivo in a suitable mammal and the monoclonal antibodies are isolated from body fluids of that mammal, and, if desired,

c) a resulting monoclonal antibody is converted into a derivative thereof.

11. A process according to claim 10, characterised in that hybridoma cells that originate from Balb/c mice and produce desired monoclonal antibodies are injected intraperitoneally into Balb/c mice that have optionally been pre-treated with a hydrocarbon, and, after 8—10 days, ascitic fluid is taken from these animals, and the monoclonal antibodies are isolated therefrom by precipitation with ammonium sulfate and purification by chromatography.

12. Hybridoma cell lines, characterised in that they produce monoclonal antibodies according to any one of claims 1 to 5.

13. The hybridoma cell line according to claim 12 designated R 3—36—16 which is deposited at the "Collection Nationale de Cultures de Microorganismes" of the Institut Pasteur in Paris under number I—253.

14. A process for the preparation of hybridoma cell lines according to claim 12, characterised in that suitable mammals are immunised with purified human renin, antibody-producing cells taken from the mammal are fused with myeloma cells, the resulting hybridoma cells are cloned and those cell clones which produce the desired monoclonal antibodies having a high affinity for human renin are selected by testing the cell supernatants for binding of monoclonal antibodies to surface-bound human renin, for binding to dissolved human renin and for inhibition of the enzyme activity of human renin.

15. A process according to claim 14, characterised in that Balb/c mice are immunised by three to six parenteral injections each containing from 1 µg to 20 µg of purified human renin, at intervals of from one to six weeks, and spleen cells are taken from the animals two to six days after the last ("booster") immunisation and fused with myeloma cells of a suitable cell line in the presence of a fusion-promoter.

16. A process according to claim 14, characterised in that monoclonal antibody-producing cells of Balb/c mice are fused with myeloma cells of the cell line X63—Ag8.653 or Sp2/O-Ag14.

17. A process according to claim 14, characterised in that myeloma cells are fused with a three- to twenty-fold excess of spleen cells from immunised animals in a solution containing from 30 to 50% polyethylene glycol having a molecular weight of from 1000 to 4000.

18. The use of monoclonal antibodies and/or their derivatives according to any one of claims 1 to 9 for the qualitative and quantitative determination of human renin and structurally similar renin *in vitro*.

19. The use of the monoclonal antibodies and derivatives according to claim 18 in a radioimmunoassay.

20. The use of the monoclonal antibodies and derivatives according to claim 18 in an enzyme-immunoassay.

21. Test kits for the determination of human renin and structurally similar renin, containing monoclonal antibodies and/or derivatives thereof according to any one of claims 1 to 9 and, optionally, adjuncts.

22. Test kits according to claim 21 for a radioimmunoassay.

23. Test kits according to claim 21 for an enzymeimmunoassay.

24. The use of monoclonal antibodies or their derivatives according to any one of claims 1 to 5 for the purification of human renin and structurally similar renin.

25. Pharmaceutical preparations containing a monoclonal antibody or a derivative thereof according to any one of claims 1 to 5 and, optionally, pharmaceutical adjuncts.

26. The use of monoclonal antibodies or derivatives thereof according to any one of claims 1 to 5 for the production of a pharmaceutical preparation for the treatment of high blood pressure and cardiac insufficiency.

## Claims for the Contracting State: AT

1. A process for the preparation of monoclonal antibodies to human renin and structurally similar renin that, at a concentration of $2 \times 10^{-8}$ M (mol/litre), inhibit the enzyme activity of human renin by at least 50%, and derivatives thereof, characterised in that hybridoma cells producing such monoclonal antibodies
   a) are cultured *in vitro* and the monoclonal antibodies are isolated from the culture supernatants, or
   b) are multiplied *in vivo* in a suitable mammal and the monoclonal antibodies are isolated from body fluids of that mammal, and, if desired,
   c) a resulting monoclonal antibody is converted into a derivative thereof.

2. A process according to claim 1, characterised in that hybridoma cells that originate from Balb/c mice and produce desired monoclonal antibodies are injected intraperitoneally into Balb/c mice that have optionally been pre-treated with a hydrocarbon, and, after 8—10 days, ascitic fluid is taken from these animals, and the monoclonal antibodies are isolated therefrom by precipitation with ammonium sulfate and purification by chormatography.

3. A process according to claim 1 for the preparation of monoclonal antibodies that, at a concentration of $3 \times 10^{-10}$ M, additionally bind at least 50% of human renin that has been added in a limiting amount.

4. A process according to claim 1 for the preparation of monoclonal antibodies that, at a concentration of $2 \times 10^{-9}$ M, inhibit the enzyme activity of human renin by at least 50%, and derivatives thereof.

5. A process according to claim 1 for the preparation of monoclonal antibodies that, at a concentration of $5 \times 10^{-11}$ M , inhibit the enzyme activity of human renin by at least 50%, and derivatives thereof.

6. A process for the preparation of monoclonal antibody R 3—36—16 produced by the hybridoma cell line designated R 3—36—16 which is deposited at the "Collection Nationale de Cultures de Microorganismes" of the Institut Pasteur in Paris under number I—253, characterised in that the hybridoma cells producing the antibodies
   a) are cultured *in vitro* and the monoclonal antibodies are isolated from the culture supernatants, or
   b) are multiplied *in vivo* in a suitable mammal and the monoclonal antibodies are isolated from body fluids of that mammal, and, if desired,

c) the resulting monoclonal antibody is converted into a derivative thereof.

7. A process according to claim 1 for the preparation of $^{125}$I-labelled monoclonal antibody derivatives, characterised in that the monoclonal antibody is reacted in step c) with $^{125}$I-sodium iodide or $^{125}$I-potassium iodide and a chemical or enzymatic oxidising agent.

8. A process according to claim 6 for the preparation of $^{125}$I-labelled derivative of the monoclonal antibody R 3—36—16.

9. A process according to claim 1 for the preparation of monoclonal antibody derivatives conjugated with alkaline phosphatase, characterised in that the monoclonal antibody is reacted in step c) with alkaline phosphatase and a coupling reagent.

10. A process according to claim 6 for the preparation of a derivative of the monoclonal antibody R 3—36—16 conjugated with alkaline phosphatase.

11. Hybridoma cell lines, characterised in that they produce monoclonal antibodies to human renin and structurally similar renin that, at a concentration of $2 \times 10^{-8}$ M, inhibit the enzyme activity of human renin by at least 50%.

12. Hybridoma cell lines according to claim 11, characterised in that they produce monoclonal antibodies that, at a concentration of $3 \times 10^{-10}$ M, additionally bind at least 50% of human renin that has been added in a limiting amount.

13. Hybridoma cell lines according to claim 11, characterised in that they produce monoclonal antibodies that, at a concentration of $2 \times 10^{-9}$ M, inhibit the enzyme activity of human renin by at least 50%.

14. Hybridoma cell lines according to claim 11, characterised in that they produce monoclonal antibodies that, at a concentration of $5 \times 10^{-11}$ M, inhibit the enzyme activity of human renin by at least 50%.

15. The hybridoma cell line designated R 3—36—16 which is deposited at the "Collection National de Cultures de Microorganismes" of the Institut Pasteur in Paris under number I—253.

16. A process for the preparation of hybridoma cell lines according to claim 11 or 15, characterised in that suitable mammals are immunised with purified human renin, antibody-producing cells taken from the mammal are fused with myeloma cells, the resulting hybridoma cells are cloned and those cell clones which produce the desired monoclonal antibodies having a high affinity for human renin are selected by testing the cell supernatants for binding of monoclonal antibodies to surface-bound human renin, for binding to dissolved human renin and for inhibition of the enzyme activity of human renin

17. A process according to claim 16, characterised in that Balb/c mice are immunised by three to six parenteral injections each containing from 1 µg to 20 µg of purified human renin, at intervals of from one to six weeks, and spleen cells are taken from the animals two to six days after the last ("booster") immunisation and fused with myeloma cells of a suitable cell line in the presence of a fusion-promoter.

18. A process according to claim 16, characterised in that monoclonal antibody-producing cells of Balb/c mice are fused with myeloma cells of the cell line X63—Ag8.653 or Sp2/O—Ag14.

19. A process according to claim 16, characterised in that myeloma cells are fused with a three- to twenty-fold excess of spleen cells from immunised animals in a solution containing from 30 to 50% polyethylene glycol having a molecular weight of from 1000 to 4000.

Figur 1

**Eichkurve zur Bestimmung von Human-Renin**

**mit dem Sandwich-RIA**

pg Human-Renin ml$^{-1}$

Figur 2    Eichkurve zur Bestimmung von Human-Renin

mit dem Sandwich-ELISA